# NEW EUROPEAN PATENT SPECIFICATION

(11) **EP 2 090 295 B2**
(45) Date of publication and mention of the opposition decision: **22.08.2018**
(45) Mention of the grant of the patent: 26.08.2015
(21) Application number: 08151246.9
(22) Date of filing: 11.02.2008
(51) Int. Cl.: A61K 8/898, A61Q 5/02, A61Q 5/06, A61Q 5/10, A61Q 5/12, A61Q 5/00

(54) **Method and composition for reducing the drying time of hair**
Verfahren und Zusammensetzung zur Verringerung der Haartrocknungszeit
Procédé et composition pour réduire le temps de séchage des cheveux

(43) Date of publication of application: 19.08.2009
(73) Proprietor: The Procter & Gamble Company, Cincinnati, OH 45202 (US)
(72) Inventor: Maillefer, Sarah, 1691 Villarimboud (CH); Pluess, Jolanda, 3175 Flamatt (CH); Flohr, Andreas, 61476, Kronberg (DE)
(74) Representative: Sauvaître, Thibault Bruno

(56) References cited:
- WO-A-2006/012930
- DE-A1-102004 030 885
- DE-A1-102004 040 266
- DE-A1-102006 032 456
- US-A- 4 563 347
- US-A- 4 586 518
- US-A- 4 973 476
- US-A- 5 567 428
- US-A1- 2001 049 849
- US-A1- 2003 053 975
- US-A1- 2004 010 863
- US-A1- 2006 041 026
- US-A1- 2006 088 490
- US-B1- 6 451 747

## Description

### FIELD OF THE INVENTION

The invention concerns to a method and composition for improving the drying time of hair, for example, after bathing, shampooing, swimming, and the like by impregnating it with a particular amino functional silicon derivative. In a preferred embodiment the invention concerns to hair styling compositions that are applied to dry or wet hair as a leave-in treatment and that additionally reduces the time to dry the hair during styling; further to leave-in or rinse-out hair care compositions that reduces the time to dry the hair.

### BACKGROUND OF THE INVENTION

In order to set and give hold to human hair in order to stabilize a created hairstyle, in order to improve the styling properties or the hair structure, hair-treatment compositions in the form of setting lotions, aerosol and non-aerosol sprays, setting foams, gels etc. are used. The cosmetic, hair-setting or haircare polymers usually used for these purposes exhibit good setting and/or care properties in aqueous, alcoholic or aqueous-alcoholic media. However, not all requirements such as, for example, gloss, shine, combability, durability of setting etc. are often still not satisfied in an entirely satisfactory manner.

It would be however especially desirable to provide further decreased drying time for hair styling compositions without any substantial loss of hair hold performance once the composition is dried to completion. It would further be desirable to even increase the hair hold performance of films formed by hair styling polymers.

To solve this problem many attempts were made to enhance the drying time of hair by adding silicone containing materials, particularly amino functional silicone polymers to the cosmetic compositions. For example it is known from the prior art, e. g. US 6451747 B1, US 4673568, US 4973476 and US 5567428 that silicone containing materials, particularly amino functional silicone polymers, known as "Amodimethicone" may be used as drying aids for cosmetic compositions.

These known silicone containing materials however display the disadvantage that the formed coating on hair is less water repellent and therefore less efficient in decreasing time to dry hair; also the coating is less resistant towards washing with shampoo and gets degraded more rapidly.

US 2006/0041026 A1 discloses an oil-in-water emulsion comprising an amino functional silicone polymer that may be used for the water repellent treatment of porous or nonporous surfaces like glass, metal, stones, paper, natural and/or synthetic textile fibers, mineral building materials, plasctics, wood, laminate, cork, rubber and leather.

One object of the present invention is to make available a cosmetic composition, preferably a hair styling composition, which enhances the drying time of hair.

A further object is to provide a new hair treatment composition that reduces the time to dry wet hair and that also style the hair.

A further object of the present invention is a hair styling composition and a method that decrease the drying time for hair styling compositions without any substantial loss of hair hold performance.

Still another object of the invention is to provide hair treatment compositions that reduce the time to dry wet hair and that also condition the hair.

It is an object of the present invention to provide a method to improve the drying of hair following showering, bathing, swimming, shampooing, or the like, by changing the hydrophobicity of the hair shaft to cause de-wetting of the hair.

A further object of the present invention is to provide a method for treating hair, preferably for styling hair, which makes the treated hair water repellent even after one hair wash, preferably even after more than 2 hair washes.

Yet another object of the invention is to provide a method of reducing damage to hair by reducing the time to dry the hair.

A further object of the invention is to improve the flexibility and manageability of hair by maintaining hair hydrated by locking water inside the hair.

A further object of the invention is to decrease the drying time for hair and simultaneously enhance the durability of a hair shape against hair humidity, water and washings.

A further object of the invention is to enhance the durability of colour of dyed hair by preventing colour molecules of dyed hair to be washed out of the hair during a hair wash.

The objects of this invention are to provide compositions and methods for fulfilling these goals. These objects and other benefits as may be apparent to those skilled in the art can be achieved through the present invention, which is described in the following Summary of the Invention and Detailed Description of the Invention and which is defined in the claims which follow.

### SUMMARY OF THE INVENTION

It has now been found that the objects are achieved by the use of a composition in the form of an emulsion as recited in claim 1, wherein the composition comprises:
(i) a polydimethylsiloxane (P) having aminoalkyl groups and having an amine number of at least 0.1 meq/g of polydimethylsiloxane (P), and comprising units of the formula

   R1ₐR²_{b}SiO_{(4-a-b)2} (I),

   in which
   R¹ are optionally halogen-substituted alkyl radicals having 1-40 carbon atoms, or are -OR or -OH radicals,
   R are optionally halogen-substituted alkyl radicals having 1-40 carbon atoms, R² are aminoalkyl radicals of the formula

      -R³-NR⁴R⁵ (II),
   R³ are divalent hydrocarbon radicals having 1-40 carbon atoms,
   R⁴ are monovalent hydrocarbon radicals having 1-40 carbon atoms or are H,
   R⁵ is a radical of the formula

      -(R⁶-NR⁴)ₓR⁴ (III),
   R⁶ is a divalent radical of the formula

      -(CR⁴R⁴-)_{y}, (IV),
   x is 0 or a value from 1 to 40,
   y is 1 or 2,
   a is 0, 1, 2 or 3,
   b is 0, 1, 2 or 3 and
   a+b on average is from 1.5 to 2.5, not more than 9 mol % of the radical R¹ being OH or OR, and (ii) a protonating agent,
   for treating human hair,
   wherein the protonating agent is formic acid, acetic acid, sulfuric acid, hydrochloric acid or citric acid;
   wherein the composition contains auxiliaries which are selected from polyalcohols and ethers thereof, which have a boiling point or boiling raise of not more than 260° C at 0.10 MPa;
   and wherein the composition contains not more than 3 parts by weight of emulsifier or surfactant.

### DETAILED DESCRIPTION OF THE INVENTION

One aspect of the invention is therefore the use of a composition in the form of an emulsion, as recited in claim 1 wherein the composition comprises:
(i) a polydimethylsiloxane (P) having aminoalkyl groups and having an amine number of at least 0.1 meq/g of polydimethylsiloxane (P), and comprising units of the formula

   R¹ₐR²_{b}SiO_{(4-a-b)'2} (I),

   in which
   R¹ are optionally halogen-substituted alkyl radicals having 1-40 carbon atoms, or are -OR or -OH radicals,
   R are optionally halogen-substituted alkyl radicals having 1-40 carbon atoms,
   R² are aminoalkyl radicals of the formula

      -R³NR⁴R⁵ (II),
   R³ are divalent hydrocarbon radicals having 1-40 carbon atoms,
   R⁴ are monovalent hydrocarbon radicals having 1-40 carbon atoms or are H,
   R⁵ is a radical of the formula

      -(R⁶-NR⁴)ₓR⁴ (III),
   R⁶ is a divalent radical of the formula

      -(CR⁴R⁴-)_{y} (IV),
   x is 0 or a value from 1 to 40,
   y is 1 or 2,
   a is 0, 1, 2 or 3,
   b is 0, 1, 2 or 3 and
   a+b on average is from 1.5 to 2.5, not more than 9 mol % of the radical R¹ being OH or OR, and (ii) a protonating agent,
   for treating human hair
   wherein the protonating agent is formic acid, acetic acid, sulfuric acid, hydrochloric acid or citric acid;
   wherein the composition contains auxiliaries which are selected from polyalcohols and ethers thereof, which have a boiling point or boiling range of not more than 260° C at 0.10 MPa;
   and wherein the composition contains not more than 3 parts by weight of emulsifier or surfactant.

The alkyl radicals R¹ and R may be linear, cyclic, branched, saturated or unsaturated. The alkyl radicals R¹ and R preferably have 1-18 carbon atoms, in particular 1-6 carbon atoms, and the methyl radical or ethyl radical is particularly preferred. Preferred halogen substituents are fluorine and chlorine. Particularly preferred radicals R¹ are the methyl radical, methoxy radical, ethoxy radical or -OH.

The divalent hydrocarbon radicals R³ may be halogen-substituted, linear, cyclic, branched, aromatic, saturated or unsaturated. The radicals R³ preferably have 1 to 6 carbon atoms, and alkylene radicals are particularly preferred, in particular propylene. Preferred halogen substituents are fluorine and chlorine.

The monovalent hydrocarbon radicals R⁴ may be halogen-substituted, linear, cyclic, branched, aromatic, saturated or unsaturated. The radicals R⁴ preferably have 1 to 6 carbon atoms, and alkyl radicals are particularly preferred. Preferred halogen substituents are fluorine and chlorine. Particularly preferred substituents R⁴ are methyl, ethyl, cyclohexyl and H.

In the polydimethylsiloxanes, b preferably has the value 0 or 1, and a+b preferably has an average value of from 1.9 to 2.2. In the polydimethylsiloxanes, x is preferably 0 or a value from 1 to 18, most preferably from 1 to 6.

Particularly preferred radicals R² are -CH₂N(R⁴)₂, -(CH₂)₃N(R⁴)₂ and -(CH₂)₃N(R⁴)(CH₂)₂N(R⁴)₂.

The polydimethylsiloxane (P) is composed of at least 3, in particular at least 10, units of the general formula (I).

The ratio of a to b is chosen so that the polydimethylsiloxane (P) has an amine number of at least 0.1 meq/g of polydimethylsiloxane (P), preferably at least 0.6 meq/g of polydimethylsiloxane (P). The amine number of the polydimethylsiloxane (P) is preferably not more than 7 meq/g of polydimethylsiloxane (P).

The viscosity of the polydimethylsiloxane (P) is preferably from 1 to 100,000 mPa·s, in particular from 10 to 10,000 mPa·s, at 25° C.

The protonating agent is formic acid, acetic acid, sulfuric acid, hydrochloric acid or citric acid. The protonating agent is preferably added in an amount of from 0.05 to 2 mol of acidic proton per mole of basic nitrogen atom of the radicals R².

The above polydimethylsiloxane (P) in combination with the protonating agent can advantageously be used for manufacturing a cosmetic composition for treating human hair.

By treating of said hair with said polydimethylsiloxane (P) together with the protonating agent the treated hair becomes water repellent. This allows to use said polydimethylsiloxane (P) and the protonating agent for reducing the drying time of human hair. This also allows to use said polydimethylsiloxane (P) and the protonating agent as disclosed above and the compositions as disclosed below, for reducing the drying time of human hair and simultaneously protect the hair dye of a previously dyed hair from being washed out.

Another object of the invention is therefore a hair treating composition, in the form of an emulsion, as recited in claim 11 comprising a combination of:
1. (A) a polydimethylsiloxane (P) having aminoalkyl groups and having an amine number of at least 0.1 meq/g of polydimethylsiloxane (P), and comprising units of the formula I

   R¹ₐR²_{b}SiO_{(4-a-b)/2} (I),

   in which
   R¹ are optionally halogen-substituted alkyl radicals having 1-40 carbon atoms, or are -OR or -OH radicals,
   R are optionally halogen-substituted alkyl radicals having 1-40 carbon atoms, R² are aminoalkyl radicals of the formula II

      -R³-NR⁴R⁵ (II),
   R³ are divalent hydrocarbon radicals having 1-40 carbon atoms,
   R⁴ are monovalent hydrocarbon radicals having 1-40 carbon atoms or are H,
   R⁵ is a radical of the formula III

      -(R⁶-NR⁴)ₓR⁴ (III),
   R⁶ is a divalent radical of the formula IV

      -(CR⁴R⁴-)_{y} (IV),
   x is 0 or a value from 1 to 40,
   y is 1 or 2,
   a is 0, 1, 2 or 3,
   b is 0, 1, 2 or 3 and
   a+b on average is from 1.5 to 2.5, not more than 9 mol % of the radical R¹ being OH or OR,
2. (B) at least one protonating agent,
3. (C) water,
4. (D) from 0 to 95 parts by weight of at least one emulsifier and/or surfactant, and
5. (E) at least one cosmetic hair treatment agent, selected from the group consisting of hair styling polymers, or hair styling polymers and hair conditioning agents,
wherein the protonatins agent is formic acid, acetic acid, sulfuric acid, hydrochloric acid or citric acid;
wherein the composition contains auxiliaries which are selected from polyalcohols and ethers thereof, which have a boiling point or boiling range of not more than 260° C at 0.10 MPa;
and wherein the composition contains not more than 3 parts by weight ofemulsifier or surfactant.

The composition is an emulsion. It is homogeneous, stable and stable to dilution without further addition of other stabilizing ingredients, such as emulsifiers or silicone/polyether copolymers. It contains not more than 3, and in particular not more than 0.5, parts by weight of emulsifier or surfactant. The composition is preferably free of an emulsifier and a surfactant.

The composition contains auxiliaries which are selected from polyalcohols and ethers thereof, which have a boiling point or boiling range of not more than 260° C at 0.10 MPa. The composition is preferably an oil-in-water emulsion.

The water in the composition of the invention is demineralized or non-demineralized water, preferably demineralized water. The water is preferably present in an amount of from 0.1 to 99 % by weight, more preferred in an amount of from 1 to 99 % by weight, more preferred in an amount of from 10 to 99 % by weight and most preferred in an amount of from 30 to 99 % by weight of the total weight of the hair treating composition.

The polydimethylsiloxanes (P) and emulsions or dispersions thereof are described in US 2006/0041026 A1 which is hereby included by reference. An oil-in-water emulsion of polydimethylsiloxanes (P) suitable for the invention is sold for example under the trademark Wacker® HC 303 VP by the company Wacker-Chemie AG, D-81737 München, Germany.

In a preferred embodiment, the composition is an emulsion and contains a MQ silicone resin. The MQ silicone resin preferably contains at least 80 mol %, preferably at least 95 mol %, of units of the general formulae (V) and (VI) R⁷₃SiO_{1/2} (V),

SiO_{4/2} (VI),

in which
R⁷ are optionally halogen-substituted hydrocarbon radicals having 1-40 carbon atoms or H, -OR or -OH radicals, and the ratio of the units of the general formulae (V) and (VI) is from 0.5 to 2.0, preferably from 0.5 to 1.5, and not more than 3% by weight, preferably not more than 2.5% by weight, of the radicals R⁷ are -OR and -OH.

The remaining units of the MQ silicone resin are preferably units of the general formulae (VII) and (VIII)

R⁷₂SiO_{2/2} (VII),

R⁷SiO_{3/2} (VIII).

The monovalent hydrocarbon radicals R⁷ may be halogen-substituted, linear, cyclic, branched, aromatic, saturated or unsaturated. The radicals R⁷ preferably have 1 to 6 carbon atoms, and alkyl radicals and phenyl radicals are particularly preferred. Further halogen substituents are fluorine and chlorine. Particularly preferred substituents R⁷ are methyl, ethyl, phenyl and H. The emulsions preferably contain from 1 to 200 parts by weight, particularly preferably from 5 to 100 parts by weight, of MQ silicone resin.

The term "hair conditioning agent" according to the present invention means any cosmetically acceptable compound having a cosmetic effect on hair, preferably selected from the group consisting of: conditioning surfactants and emulsifiers, conditioning thickeners, silicon compounds, oils, waxes, fatty alcohols, hydrogenated fats, fatty acid esters, conditioning thickeners, lanoline, amino acids, casein, ceresin, paraffines, betaine, panthenol, sorbitol, protein hydrolysates, ceramids, plant extracts, zein, silk, squalene, urea and hinokitiol.

Preferrred hair conditioning agents are paraffin waxes, polyolefin waxes, wool wax, wool wax alcohols, candelilla wax, olive wax, carnauba wax, Japan wax, apple wax, hydrogenated fats, fatty alcohols, fatty acid esters, fatty acid glycerides, fatty acid triglycerides, conditioning emulsifiers, conditioning surfactants, conditioning thickeners, mineral oils, isoparaffin oils, paraffin oils, squalane, sunflower oil, coconut oil, castor oil, lanolin oil, jojoba oil, corn oil or soybean oil.

This is further specified thereafter.

In another aspect of the present invention there is to provide a composition and method for styling the hair while simultaneously reducing the time to dry the hair. As previously recited, the method for reducing the time to dry wet hair, for example, drying wet hair following showering, shampooing, bathing, swimming, and the like, comprises applying to wet hair an aqueous, aqueous-alcoholic or aqueous-organic-solvent hair styling composition of the invention, a styling component, water and optionally another solvent, and subsequently drying the hair. Before or during the drying step, the hair may be mechanically processed to help water to sheet from the hair. Such processing includes brushing, combing, towelling, and the like.

In the case of towelling, it is seen that mechanical action is typically combined with absorbing of the water present on the hair. In this embodiment, the composition of the invention in an aqueous or partially aqueous vehicle. Surprisingly, the time to dry the hair is reduced, notwithstanding the application to hair of additional water present in the composition.

The composition according to the invention can be used in various application forms, such as, for example, as lotion, as non-aerosol spray lotion, which is used by means of a mechanical device for spraying, as aerosol spray which is sprayed by means of a propellant, as aerosol foam or as non-aerosol foam which is present in combination with a suitable mechanical device for foaming the composition, as a mousse, as hair cream, as hair wax, as gel, as liquid gel, as sprayable gel or as foam gel, pump, putty, spray wax, pomade, brilliantine, balm, stick, strip, dispersion or emulsion. A use in the form of a lotion thickened with a customary thickener is also possible.

The composition of the invention has preferably the form of an oil-in-water emulsion.

The products are leave-in products that are applied to wet or dry hair, any may be used to treat the hair, for example, styling products and fixative products or products to condition hair.

The term "hair conditioning agent" according to the present invention means an agent that provides gloss to hair, makes hair more manageable, improve hair touch, improve combability and/or give hair more volume.

In the composition according to the invention the polydimethylsiloxane (P) is preferably present in an amount of from 0.001 to 50 % by weight, more preferred in an amount of from 0.01 to 30 % by weight, still more preferred in an amount of from 0.1 to 20 %, still more preferred in an amount of from 0.1 to 10 % and most preferred in an amount of from 0.1 to 5 % by weight of the total weight of the hair treating composition.

When the composition according to the invention is a hair styling composition it preferably comprises from 0.1 to 10 % by weight, more preferred from 0.5 to 8 % and most preferred from 1 to 5 % by weight of at least one hair styling polymer.

### The Hair Treatment Agent

The hair treatment agents for use in the hair treatment compositions of the present invention are hair styling polymers, or hair styling polymers and hair conditioning agents. The hair treatment agents permit the consumer to style the hair in conjunction with combing or brushing, and especially coincidental to drying with a blow dryer or other device that supplies heat to the hair. The hair treatment compositions are especially applied to damp hair.

The hair fixative agents are preferably nonionic polymers, but may also be cationic, anionic, and amphoteric polymers. Suitable hair fixative agents are identified in the International Cosmetic Ingredient Dictionary and Handbook, v. 3, p. 2227-8 (10th Edition 2004) published by the Cosmetics, Toiletries and Fragrance Association (CTFA) (hereinafter referred to as "INCI").

The hair conditioning agents are typically cationic polymers that provide softness to the hair or that repair damaged hair. The conditioning polymers generally provide a film on the hair that is smooth and not tacky.

Typically, the conditioning polymers are cationic but may also be nonionic, zwitterionic, and amphoteric. The hair conditioning agents may also be oils, as described in greater detail below.

As known in the art, there is some overlap between polymers that provide hair conditioning benefit and hair fixative benefits, depending often on the concentration of the polymer and the other components present in the formulation.

### HAIRSTYLING POLYMERS

### Nonionic hair styling polymers

Suitable synthetic, nonionic film-forming, hair-styling polymers are homopolymers or copolymers which are constructed from at least one of the following monomers: vinylpyrrolidone, vinylcaprolactam, vinyl esters, such as, for example, vinyl acetate, vinyl alcohol, acrylamide, methacrylamide, alkyl- and dialkylacrylamide, alkyl- and dialkylmethacrylamide, alkyl acrylate, alkyl methacrylate, propylene glycol or ethylene glycol, where the alkyl groups of these monomers are preferably C1- to C7-alkyl groups, particularly preferably C1- to C3-alkyl groups. Homopolymers of vinylcaprolactam, of vinylpyrrolidone or of N-vinylformamide, for example are suitable. Further suitable synthetic film-forming, nonionic, hair-setting polymers are, for example, copolymers of vinylpyrrolidone and vinyl acetate, terpolymers of vinylpyrrolidone, vinyl acetate and vinyl propionate, polyacrylamide; polyvinyl alcohols, and polyethylene glycol/polypropylene glycol copolymers. Suitable natural film-forming polymers are, for example, cellulose derivatives, e.g. hydroxyalkylcellulose.

Among the suitable nonionic hairstyling polymers, mention may be made of homo- and copolymers of vinylpyrrolidone, especially, copolymers of vinylacetate, in particular those sold under the trade name Luviskol®, e.g. homopolymers Luviskol® K 30, K 60 or K 90; copolymers Luviskol® VA 55, VA 64; and the terpolymer vinylpyrrolidone/vinylacetate/vinylpropionate copolymer sold as Luviskol® V AP 343 all from BASF; terpolymer of vinylpyrrolidone, methacrylamide and vinylimidazole (Luviset® Clear of BASF); vinyl pyrrolidone-/acrylates copolymer; vinyl pyrrolidone/hexadecane copolymer, and vinyl pyrrolidone/vinyl caprolactam/dimethylaminoethyl methacrylate acrylates copolymer.

Other nonionic hairstyling polymers suitable as the hair fixative agent include VP/acrylates/lauryl methacrylate copolymer; adipic acid/diethylenetriamine copolymer; PEG-8/SMDI copolymer; polyacrylamide-1; polyvinyl acetate; PPG-12/SMDI copolymer; polyurethane-1, polyurethane-14; and polyimide-1 sold as Aquaflex XL-30 by ISP.

Suitable hairstyling polymers with acid groups are, in particular, homopolymers of acrylic acid or of methacrylic acid which are uncrosslinked or crosslinked with polyfunctional agents, copolymers of acrylic acid or methacrylic acid with monomers chosen from acrylic or methacrylic esters, acrylamides, methacrylamides and vinylpyrrolidone, homopolymers of crotonic acid, and copolymers of crotonic acid with monomers chosen from vinyl esters, acrylic or methacrylic esters, acrylamides and methacrylamides. A suitable natural polymer is, for example, Shellac.

### Preferred nonionic hairstyling polymers are:

Polyvinylpyrrolidone, polyvinylcaprolactam, vinylpyrrolidone/vinyl acetate copolymers, polyvinyl alcohol, isobutylene/ethylmaleimide/hydroxy-ethylmaleimide copolymer; copolymers of vinylpyrrolidone, vinyl acetate and vinyl propionate. Further preferred polymers are described in WO 03/092640, e. g. example Nos. 61, 62, 64 and 65, and are commercially available under the name Luviflex® Clear (BASF) (INCI name: VP/Methacrylamide/Vinyl Imidazole Copolymer).

### Anionic hairstyling polymers

### Preferred hairstyling polymers with acid groups are:

Terpolymers of acrylic acid, alkyl acrylate and N-alkylacrylamide (INCI name: Acrylates/Acrylamide Copolymer), in particular terpolymers of acrylic acid, ethyl acrylate and N-tert-butylacrylamide; crosslinked or uncrosslinked vinyl acetate/crotonic acid copolymers (INCI name: VA/Crotonates Copolymer); copolymers of one or more C1-C5-alkyl acrylates, in particular C2-C4-alkyl acrylates and at least one monomer chosen from acrylic acid or methacrylic acid (INCI name: Acrylates Copolymer), e.g. terpolymers of tert-butyl acrylate, ethyl acrylate and methacrylic acid; sodium polystyrenesulfonate; vinyl acetate/crotonic acid/vinyl alkanoate copolymers, e.g. copolymers of vinyl acetate, crotonic acid and vinyl propionate; copolymers of vinyl acetate, crotonic acid and vinyl neodecanoate (INCI names: VA/Crotonates/Vinyl Propionate Copolymer, VA/Crotonates/Vinyl Neodecanoate Copolymer); aminomethylpropanol acrylate copolymers; copolymers of vinylpyrrolidone and at least one further monomer chosen from acrylic acid and methacrylic acid, and optionally acrylic esters and methacrylic esters; copolymers of methyl vinyl ether and maleic monoalkyl esters (INCI names: Ethyl ester of PVM/MA Copolymer, Butyl ester of PVM/MA Copolymer); aminomethylpropanol salts of copolymers of allyl methacrylate and at least one further monomer chosen from acrylic acid, and methacrylic acid, and optionally acrylic esters and methacrylic esters; crosslinked copolymers of ethyl acrylate and methacrylic acid; copolymers of vinyl acetate, mono-n-butyl maleate and isobornyl acrylate; copolymers of two or more monomers chosen from acrylic acid and methacrylic acid, and optionally acrylic esters and methacrylic esters; copolymers of octylacrylamide and at least one monomer chosen from acrylic acid and methacrylic acid, and optionally acrylic esters and methacrylic esters; polyesters of diglycol, cyclohexanedimethanol, isophthalic acid and sulfoisophthalic acid, where the alkyl groups of the abovementioned polymers generally have preferably 1, 2, 3 or 4 carbon atoms.

### Preferred zwitterionic or amphoteric hairstyling polymers are:

Copolymers formed from alkylacrylamide, alkylaminoalkyl methacrylate and two or more monomers of acrylic acid and methacrylic acid, and optionally esters thereof, in particular copolymers of octylacrylamide, acrylic acid, butylaminoethyl methacrylate, methyl methacrylate and hydroxypropyl methacrylate (INCI name:
Octylacrylamide/Acrylates/Butylaminoethyl Methacrylate Copolymer); copolymers which are formed from at least one first monomer type which has quaternary amine groups, and at least one second monomer type which has acid groups; copolymers of fatty alcohol acrylates, of alkylamine oxide methacrylate and at least one monomer chosen from acrylic acid and methacrylic acid, and optionally acrylic esters and methacrylic esters, in particular copolymers of lauryl acrylate, stearyl acrylate, ethylamine oxide methacrylate and at least one monomer chosen from acrylic acid and methacrylic acid, and optionally esters thereof;
copolymers of methacryloylethylbetaine and at least one monomer chosen from methacrylic acid and methacrylic esters; copolymers of acrylic acid, methyl acrylate and methacrylamidopropyl-trimethylammonium chloride (INCI name: Polyquaternium-47);
copolymers of acrylamidopropyltrimethylammonium chloride and acrylates or copolymers of acrylamide, acrylamidopropyltrimethylammonium chloride, 2-amido-propylacrylamidesulfonate and dimethylaminopropylamine (INCI name: Polyquaternium-43);
oligomers or polymers, preparable from quaternary croton betaines or quaternary croton betaine esters.

### Cationic hairstyling polymers

The cationic polymers may be homopolymers or copolymers, where the quaternary nitrogen groups are present either in the polymer chain or preferably as substituent on one or more of the monomers. The monomers containing ammonium groups may be copolymerized with noncationic monomers. Suitable cationic monomers are unsaturated, free-radically polymerizable compounds which carry at least one cationic group, in particular ammonium-substituted vinyl monomers, such as, for example, trialkylmethacryloxyalkylammonium, trialkylacryloxyalkylammonium, dialkyldiallylammonium and quaternary vinylammonium monomers with cyclic, cationic nitrogen-containing groups, such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups, such as, for example, C1- to C7-alkyl groups, particularly preferably C1- to C3-alkyl groups.

The monomers containing ammonium groups may be copolymerized with noncationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, e.g. vinyl acetate, vinyl alcohol, propylene glycol or ethylene glycol, where the alkyl groups of these monomers are preferably C1- to C7-alkyl groups, particularly preferably C1- to C3-alkyl groups.

Suitable hairstyling polymers with quaternary amine groups are, for example, the polymers described in the CTFA Cosmetic Ingredient Dictionary under the names Polyquaternium, such as methylvinylimidazolium chloride/vinylpyrrolidone copolymer (Polyquaternium-16) or quaternized vinylpyrrolidone/dimethylaminoethyl methacrylate copolymer (Polyquaternium-11), and quaternary silicone polymers and oligomers, such as, for example, silicone polymers with quaternary end groups (Quaternium-80).

### Preferred synthetic-based cationic hair styling polymers:

Poly(dimethyldiallyammonium chloride); copolymers of acrylamide and dimethyldiallylammonium chloride; quaternary ammonium polymers formed by the reaction of diethyl sulfate and a copolymer of vinylpyrrolidone and dimethylaminoethyl methacrylate, in particular vinylpyrrolidone/dimethyl-laminoethyl methacrylate methosulfate copolymer (e.g. Gafquat® 755 N, Gafquat® 734); quaternary ammonium polymers of methylvinylimidazolium chloride and vinylpyrrolidone (e.g. LUVIQUAT® HM 550); Polyquaternium-35; Polyquaternium-57; polymer of trimethylammonium ethyl methacrylate chloride; terpolymers of dimethyldiallylammonium chloride, sodium acrylate and acrylamide (e.g. Merquat® Plus 3300); copolymers of vinylpyrrolidone, dimethylaminopropyl methacrylamide and methacryloylaminopropyllauryl-dimethylammonium chloride; terpolymers of vinylpyrrolidone, dimethylaminoethyl methacrylate and vinylcaprolactam (e.g. Gaffix® VC 713); vinylpyrrolidone/methacrylamidepropyltrimethylammonium chloride copolymers (e.g. Gafquat® HS 100); copolymers of vinylpyrrolidone and dimethylaminoethyl methacrylate; copolymers of vinylpyrrolidone, vinylcaprolactam and dimethylaminopropylacrylamide; polyesters or oligoesters constructed from at least one first monomer type which is chosen from hydroxy acid substituted by at least one quaternary ammonium group; dimethylpolysiloxanes terminally substituted by quaternary ammonium groups.

Preferred hairstyling polymers with amino groups are copolymers of vinylpyrrolidone, vinylcaprolactam and a basic acrylamide monomer, where the basic acrylamide monomer is preferably chosen from dialkylaminoalkyl methacrylamide and dialkylaminoalkyl acrylamide, where the alkyl groups consist of 1 to 4 carbon atoms. Particular preference is given to dimethylaminopropyl methacrylamide. The preparation of such a polymer is described in WO 96/19971 and is commercially available under the name Aquaflex® SF 40 (ISP).

Suitable cationic hairstyling polymers which are derived from natural polymers are, in particular, cationic derivatives of polysaccharides, for example cationic derivatives of cellulose, starch or guar. Also suitable are chitosan and chitosan derivatives. Cationic polysaccharides have, for example, the general formula

G-O-B-N⁺R^{a}R^{b}R^{c} X⁻

G is an anhydroglucose radical, for example starch or cellulose anhydroglucose; B is a divalent compound group, for example alkylene, oxyalkylene, polyoxyalkylene or hydroxyalkylene;
R^{a}, R^{b} and R^{c}, independently of one another, are alkyl, aryl, alkylaryl, arylalkyl, alkoxyalkyl or alkoxyaryl in each case having up to 18 carbon atoms, where the total number of the carbon atoms in R^{a}, R^{b} and R^{c} is preferably at least 20; X is a customary counteranion, for example a halogen, acetate, phosphate, nitrate or alkyl sulfate, preferably a chloride.

Cationic celluloses are, for example, those with the INCI names Polyquaternium-10 or Polyquaternium-24. A suitable cationic guar derivative has, for example, the INCI name Guar Hydroxypropyltrimonium Chloride.

Preferred cationic cellulose compounds are those which have at least one quaternary ammonium group, e.g. a copolymer of hydroxyethylcellulose and diallyldimethylammonium chloride (Polyquaternium-4) or the reaction product of hydroxyethylcellulose and an epoxide substituted by a trialkylammonium group (Polyquaternium-10), where the alkyl groups can have from 1 to 20 carbon atoms and are preferably methyl groups. The molecular weight is preferably between 100 000 and 600 000, particularly preferably between 200 000 and 400 000. The nitrogen content is preferably 0.5 to 4%, particularly preferably 1.5 to 3%. A preferred cellulose derivative is Polyquaternium-4, which is sold under the trade names Celquat® H100 and Celquat® L200, of which Celquat® L200 is particularly preferred.

Particularly preferred cation-active hairstyling polymers are chitosan, chitosan salts and chitosan derivatives. The chitosans to be used according to the invention are completely or partially deacetylated chitins. The molecular weight can be spread over a broad spectrum, for example from 20 000 to about 5 million g/mol, e.g. from 30 000 to 70 000 g/mol. Preferably, however, the molecular weight is more than 100 000 g/mol, particularly preferably from 200 000 to 700 000 g/mol. The degree of deacetylation is preferably 10 to 99%, particularly preferably 60 to 99%. A preferred chitosan salt is chitosonium pyrrolidonecarboxylate, e.g. Kytamer® PC with a molecular weight of from about 200 000 to 300 000 g/mol and deacetylation of from 70 to 85%. Suitable chitosan derivatives are quaternized, alkylated or hydroxyalkylated derivatives, e.g. hydroxyethyl-, hydroxypropyl- or hydroxybutylchitosan. The chitosans or chitosan derivatives are preferably in neutralized or partially neutralized form. The degree of neutralization is preferably at least 50%, particularly preferably between 70 and 100%, based on the number of free base groups. Neutralizing agents which can be used are, in principle, all cosmetically compatible inorganic or organic acids, such as, for example, formic acid, tartaric acid, malic acid, lactic acid, citric acid, pyrrolidonecarboxylic acid, hydrochloric acid etc., of which pyrrolidonecarboxylic acid is particularly preferred.

### Preferred natural-based cationic hairstyling polymers:

Cationic cellulose derivatives of hydroxyethylcellulose and diallyldimethylammonium chloride; cationic cellulose derivatives of hydroxyethylcellulose and epoxide substituted by trimethylammonium; chitosan and salts thereof; hydroxyalkylchitosans and salts thereof; alkylhydroxyalkylchitosans and salts thereof; N-hydroxyalkylchitosan alkyl ethers; N-hydroxyalkylchitosan benzyl ethers.

In a further preferred embodiment, the composition according to the invention comprises 0.01 to 15% by weight, preferably 0.5 to 10% by weight, of at least one synthetic or natural nonionic film-forming polymer. Natural polymers are also understood as meaning chemically modified polymers of natural origin. Film-forming polymers are understood as meaning those polymers which, when used in 0.01 to 5% strength aqueous, alcoholic or aqueous-alcoholic solution, are able to deposit a polymer film on the hair.

If the hair treating composition is a hair styling composition, the hair styling polymer is present in an amount of from 0.1 to 10% by weight and preferred in an amount of from 0.5 to 6 % by weight.

If the hair treating composition is a hair care composition, the hair styling polymer is preferably present in an amount of from 0.01 to 3 % by weight, and more preferred in an amount of from 0.1 to 2 % by weight.

### HAIR CONDITIONING AGENTS

### Cationic Surfactants

Hair conditioning agents in form of cationic surfactants that can be preferably used in the cosmetic composition of the present invention contain amino or quaternary ammonium moieties.

Among the quaternary ammonium-containing cationic surfactant materials useful herein are those of the general formula

[NR4,R5,R6,R7]⁺. X⁻

wherein R4 to R7 are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X⁻ is a salt-forming anion such as those selected from halogen, (e. g. chloride, bromide, iodide), acetate, citrate, lactate, glycolate, phosphate nitrate, sulfate, and alkylsulfate radicals.

The aliphatic groups may contain, in addition to carbon and hydrogen atoms, either linkages, and other groups such as amino groups. The longer chain aliphatic groups, e. g. those of about 12 carbons, or higher, can be saturated or unsaturated. Especially preferred are di-long chain (e. g. di C12-C22, preferably C16-C18, aliphatic, preferably alkyl) and di-short chain (e. g. C1 - C3 alkyl, preferably C1 - C2 alkyl) ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydorxy ethyl stearylamine, and arachidylbehenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Preferred cationic surfactants are cetyl trimethyl ammonium salts, as for example Genamin® CTAC, i. e. cetyl trimethyl ammonium chloride, behenyl trimethyl ammonium salts, e. g. behenyl trimethyl ammonium chloride; dimethyl ditallow ammonium salts; stearyl amidopropyl dimethylamine; esterquats as for example tetradecyl betainester chloride, diesterquats as for example dipalmitylethyl - dimethylammoniumchloride (Armocare® VGH70 of Akzo, Germany), or a mixture of distearoylethyl hydroxyethylmonium methosulfate and Cetearyl Alkohol (Dehyquart® F-75 of Henkel, Germany).

Preferred cationic surfactants include QUATERNIUM-8; QUATERNIUM-14; QUATERNIUM-15; QUATERNIUM-18; QUATERNIUM-22; QUARTERNIUM-24; QUATERNIUM-26; QUATERNIUM-27; QUATERNIUM-30; QUATERNIUM-33; QUATERNIUM-37; QUATERNIUM-53; QUATERNIUM-60; QUATERNIUM-61; QUATERNIUM-72; QUATERNIUM-78; QUATERNIUM-80; QUATERNIUM-81; QUATERNIUM-82; QUATERNIUM-83; QUATERNIUM-84; AND QUATERNIUM-91.

### Nonionic Surfactants

Hair conditioning agents in form of nonionic surfactants that can be used in the cosmetic composition of the present invention are selected from the group of nonionic surfactants having an HLB of less than 8.

The nonionic surfactant of HLB < 8 can preferably be chosen in particular from glyceryl esters, such as mono-, di- or triglyceryl mono-, di- or triisostearate or -oleate, sugar esters, such as sucrose or methyl glucose mono- or diisostearate or -oleate, alkylpolyglucoside ethers, such as sorbitan isostearate, oleyl- or isostearylpolyglucoside; polyoxyethylene (20) sorbitan monostearate (CTFA: Polysorbat-60), and their mixtures. Sugar esters and alkylpolyglucoside ethers are most preferred.

### Silicone Conditioning Agents

The cosmetic compositions hereof can also include volatile or nonvolatile, soluble or insoluble silicones as conditioning agents. By soluble what is meant is that the silicone conditioning agent is miscible with the aqueous carrier of the composition so as to form part of the same phase. By insoluble what is meant is that the silicone forms a separate, discontinuous phase from the aqueous carrier, such as in the form of an emulsion or a suspension of droplets of the silicone.

Soluble silicones include silicone copolyols, such as dimethicone copolyols, e.g. polyether siloxane-modified polymers, such as polypropylene oxide, polyethylene oxide modified polydimethylsiloxane, wherein the level of ethylene and/or propylene oxide sufficient to allow solubility in the composition.

Preferred silicones are: Cyclic dimethylsiloxanes, linear polydimethylsiloxanes, block polymers of polydimethylsiloxane and polyethylene oxide and/or polypropylene oxide, polydimethylsiloxanes with terminal or lateral polyethylene oxide or polypropylene oxide radicals, polydimethylsiloxanes with terminal hydroxyl groups, phenyl-substituted polydimethylsiloxanes, silicone emulsions, silicone elastomers, silicone waxes, silicone gums and amino-substituted silicones.

Preferred, however, are insoluble silicones. The insoluble silicone hair conditioning agent for use herein will preferably have viscosity of from about 1,000 to about 2,000,000 mPa· s at 25°C, more preferably from about 10,000 to about 1,800,000, even more preferably from about 100,000 to about 1,500,000 mPa· s at 25°C. The viscosity can be measured by means of a glass capillary viscometer as set forth in Dow Corning Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof. Specific examples thereof include hexamethyldisiloxane, octamethyltrisiloxane, decamethyltetrasiloxane and hexadecamethylheptasiloxane.

Other insoluble, nonvolatile silicone fluids having hair conditioning properties can also be used. The term "nonvolatile" as used herein shall mean that the silicone has a boiling point of at least about 260°C, preferably at least about 275°C, more preferably at least about 300°C. Such materials exhibit very low or no significant vapor pressure at ambient conditions. The term "silicone fluid" shall mean flowable silicone materials having a viscosity of less than 1,000,000 mPa· s at 25°C. Generally, the viscosity of the fluid will be between about 5 and 1,000,000 mPa· s at 25°C, preferably between about 10 and about 300,000 mPa· s at 25°.

The preferred silicones are polydimethyl siloxane, polydiethylsiloxane, and polymethylphenylsiloxane. Polydimethylsiloxane is especially preferred. The nonvolatile polyalkylsiloxane fluids that may be used include, for example, polydimethyl siloxanes. These siloxanes are available, for example, from the General Electric Company in their Viscasil R and SF 96 series, and from Dow Corning in their Dow Corning 200® series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

Suitable insoluble, volatile silicone fluids include low molecular weight oligomeric polydimethylsiloxane or cyclic polydimethylsiloxane, having a viscosity of no more than 10 mPa· s at 25°C and a boiling point under atmospheric pressure of no more than 250°C. Volatility can be achieved in linear organopolysiloxanes by selection of oligomeric organopolysiloxanes with at most 6 to 10 silicone atoms in the organopolysiloxanes backbone, e. g. Dow Corning DC200 Fluid, having a viscosity of from about 0.65 to about 2 mPa· s at 25°C. Preferably cylclic organopolysiloxanes having from 3 to 6 silicon atoms are utilized, for example, hexamethylcyclotrisiloxane, octamethylcyclotetrasiloxane, decamethylcyclopentasiloxane, dodecamethylcyclohexasiloxane (e. g. DC 244, DC 245, DC 345 of Dow Corning).

Especially preferred, for enhancing the shine characteristics of hair, are highly arylated silicones, such as highly phenylated polyethyl silicone having refractive indices of about 1.46 or higher, especially about 1.52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e. g. Dow Corning DC-1248®) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 mPa· s. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof. Preferably the silicone hair treating agent comprises a mixture of a polydimethylsiloxane gum, having a viscosity greater than about 1,000,000 mPa · s and polydimethylsiloxane fluid having a viscosity of from about 10 mPa· s to about 100,000 mPa· s at 25°C, wherein the ratio of gum to fluid is from about 30:70 to about 70:30, preferably from about 40:60 to about 60:40.

Silicone conditioning polymers that are specially preferred are CTFA: DIMETHICONE BISAMINO HYDROXYPROPYL COPOLYOL, BISAMINO PEG/PPG-41/3 AMINOETHYL PG-PROPYL DIMETHICONE, dihydroxypoly-dimethylsiloxane (CTFA: DIMETHICONOL). Also preferred are volatile silicones such as e. g. CTFA: DIMETHICONE, DIMETHICONE COPOLYOL and CYCLOMETHICONE.

The silicone hair conditioning agent can be used in the compositions hereof at levels of from about 0.1 % to about 20 % by weight of the composition, preferably from about 0.1 % to about 10%, more preferably from about 0.1 % to about 5% and most preferably from about 0.2 % to about 1 % by weight.

### Organic Oily Conditioning Agents

The compositions of the present invention can also comprise a nonvolatile, water insoluble, organic, oil or fat as a conditioning agent for hair or skin.

The hair conditioning oil can add shine and luster to the hair. The conditioning oil is typically present in the compositions at a level of from about 0.05% to about 5%, by weight of the composition, preferably from about 0.2% to about 3%, more preferably from about 0.5% to about 1%.

By "nonvolatile" what is meant is that the oily material exhibits very low or no significant vapor pressure at ambient conditions (e.g., 1 atmosphere, 25° C.), as is understood in the art. The nonvolatile oily materials preferably have a boiling point at ambient pressure of about 250° C. or higher.

By "water insoluble" what is meant is that the oily liquid is not soluble in water (distilled or equivalent) at a concentration of 0.1 %, at 25° C.

The conditioning oils hereof are liquids selected from the group consisting of hydrocarbon oils and fatty esters. The fatty esters hereof are characterized by having at least 10 carbon atoms, and include esters with hydrocarbyl chains derived from fatty acids or alcohols, e.g. mono-esters, polyhydric alcohol esters, and di- and tri-carboxylic acid esters. The hydrocarbyl radicals of the fatty esters hereof can also include or have covalently bonded thereto other compatible functionalities, such as amides and alkoxy moieties (e.g., ethoxy or ether linkages, etc.).

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 19 carbon atoms, although it is not necessarily meant to be limit the hydrocarbons to this range. Branched chain hydrocarbon oils can and typically may contain higher numbers of carbon atoms. Also encompassed herein are polymeric hydrocarbons of alkenyl monomers, such as C₂ -C₆ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 500, preferably from about 200 to about 400, more preferably from about 300 to about 350.

Specific examples of suitable materials include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A preferred hydrocarbon polymer is polybutene, such as the copolymer of isobutylene and butene. A commercially available material of this type is L-14 polybutene from Amoco Chemical Co. (Chicago, III., U.S.A.).

Monocarboxylic acid esters hereof inlude esters of alcohols and/or acids of the formula R'COOR wherein alkyl or alkenyl radicals and the sum of carbon atoms in R' and R is at least 10, preferably at least 20.

Fatty esters include, for example, alkyl and alkenyl esters of fatty acids having aliphatic chains with from about 10 to about 22 carbon atoms, and alkyl and alkenyl fatty alcohol carboxylic acid esters having an alkyl and/or alkenyl alcohol-derived aliphatic chain with about 10 to about 22 carbon atoms, and combinations thereof.

Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

The mono-carboxylic acid ester however need not necessarily contain at least one chain with at least 10 carbon atoms, so long as the total number of aliphatic chain carbon atoms is at least 10. Examples include diisopropyl adipate, diisohexyl adipate, and diisopropyl sebacate.

Di- and tri-alkyl and alkenyl esters of carboxylic acids can also be used. These include, for example, esters of C₄ -C₈ dicarboxylic acids such as C₁ - C₂₂ esters (preferably C₁ -C₆) of succinic acid, glutaric acid, adipic acid, hexanoic acid, heptanoic acid, and octanoic acid. Specific examples include isocetyl stearyol stearate, diisopropyl adipate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters for use herein.

Glycerides include mono-, di-, and tri-glycerides. More specifically, included are the mono-, di-, and tri-esters of glycerol and long chain carboxylic acids, such as C₁₀ -C₂₂ carboxylic acids. Synthetic oils include triolein and tristearin glyceryl dilaurate. Preferred glycerides are di-, and tri-glycerides. Especially preferred are triglycerides.

A variety of these types of materials can be obtained from vegetable and animal fats and oils, such as castor oil, safflower oil, sunflower oil, cottonseed oil, corn oil, olive oil, cod liver oil, almond oil, palm oil, sesame oil, lanolin and soybean oil, avocado oil, tsubaki oil, turtle oil, macademia nuts oil, corn oil, mink oil, olive oil, rape seed oil, yolk oil, sesame oil, parsic oil, wheat germ oil, sasanqua oil, linseed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, tallow, sheep fat, hardened tallow, palm kernel oil, jojoba oil, lard, ox bone fat, wood wax kernel oil, hardened castor oil; waxes such as beeswax, apple wax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons , nonvolatile hydrocarbons and hydrocarbon esters such as fluid paraffin, solid paraffin, vaseline, ozocerite, squalane, pristan, ceresin, squalane, petrolatum, isododecane, microcrystalline wax; fatty acid oils, ester oils such as cetyl octanoate, isopropyl myristate; betaine, carnitin, carnitin esters, creatine, amino acids, peptides, proteines, vitamines, phospholipides, e. g. lecithines or ceramides. Useful are also quaternary benzyl salts such as lauryl-dimethyl-ethylbenzyl-ammonium chloride (QUATERNIUM-14); imidazolidinyl derivatives as for example CTFA: QUATERNIUM-87 (Rewoquat® W 575 of Witco, Germany); N-(3-chloroallyl)hexaminium chloride (QUARTERNIUM-15); a quaternary ammonium compound sold under the trade name Finquat CT by the company Finetex (CTFA: QUATERNIUM-75; see CTFA Tenth Edition, page 1604).

The amount of the above conditioning agents preferably ranges from about 0.1 % to 30% by weight, better still from about 0.1 % to 15% by weight and most preferred from about 0.2 % to 10% by weight of the total weight of the cosmetic composition. These ranges include all specific values and sub-ranges there between, including 3, 8, 10, 15, 20, 25 and 30% by weight.

### Fatty Alcohols

The cosmetic compositions of the present invention may also comprise as hair conditioning agent a nonvolatile low melting point fatty alcohol. The fatty alcohols hereof have a melting point of 30°C or less, preferably about 25°C or less, more preferably about 22°C or less. The unsaturated fatty alcohols hereof are also nonvolatile. By nonvolatile what is meant is they have a boiling point at 1.0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, preferably less than about 5% by total weight of the unsaturated straight chain fatty alcohol more preferably less than about 2%, most preferably less than about 1%. Preferably, the unsaturated straight chain fatty alcohols will have an aliphatic chain size of from C12-C22, more preferably from C12-C18, most preferably from C16-C18. Exemplary alcohols of this type include oleyl alcohol, and palmitoleic alcohol. The branched chain alcohols will typically have aliphatic chain sizes of from C12-C22, preferably C14-C20, more preferably C16-C18.

Exemplary branched chain alcohols for use herein include stearyl alcohol, cetyl alcohol, isostearyl alcohol, octyl dodecanol, and octyl decanol.

Examples of saturated C8-C12 straight chain alcohols include octyl alcohol, caprylic alcohol, decyl alcohol, and lauryl alcohol. The low melting point fatty alcohols hereof are used at a level of from about 0.1 % to about 15 %, by weight of the composition, more preferably from about 0.2 % to about 11 %, most preferably from about 0.2 % to about 3 %.

It may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10.

The total amount of fatty alcohols in the composition is preferably about 0.5 to about 15.0 % by weight, more preferably from about 1.0 to about 12.0 % by weight, and most preferably from about 1.5 to about 11.0 % by weight.

### OTHER INGREDIENTS

The cosmetic compositions herein can contain a variety of other optional components suitable for rendering such compositions more cosmetically or aesthetically acceptable or to provide them with additional usage benefits. Such conventional optional ingredients are well-known to those skilled in the art. The composition of the invention may thus comprise lipophilic or hydrophilic adjuvants which are standard in the cosmetics or dermatological fields, such as surfactants, in particular foaming surfactants, preservatives, antioxidants, sequestering agents, solvents, fragrances, fillers, screening agents, odor absorbers, coloring materials and lipid vesicles.

A wide variety of additional ingredients can be formulated into the present cosmetic composition. These include: detersive surfactants such as anionic, nonionic, amphoteric, and zwitterionic surfactants; additional thickening agents and suspending agents, such as xanthan gum, guar gum, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; UV-filters and sunscreens, e. g. such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; agents for combating free radicals; preservatives such as benzyl alcohol, methyl paraben, propyl paraben and imidazolidinyl urea; polyvinyl alcohol; pH adjusting agents, such as citric acid, formic acid, glyoxylic acid, acetic acid, lactic acid, pyruvic acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes, sequestering agents, such as disodium ethylenediamine tetra-acetate, and polymer plasticizing agents, such as glycerin, disobutyl adipate, butyl stearate, and propylene glycol.

### Solvents

The composition according to the invention is preferably formulated in as aqueous, alcoholic or aqueous-alcoholic composition with preferably at least 10% by weight of water. The hair treating composition can therefore optionally include liquid water-miscible or water-soluble solvents such as lower alkyl alcohols, e. g. C₁-C₅ alkyl monohydric alcohols, preferably C₂-C₃ alkyl alcohols. Alcohols which may be present are in particular lower monohydric or polyhydric alcohols having 1 to 4 carbon atoms customarily used for cosmetic purposes, such as preferably ethanol and isopropanol.

The water-soluble polyhydric alcohols usable in the present invention are also polyhydric alcohols having two or more hydroxyl groups in the molecule. Typical examples of such polyhydric alcohols are dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether.

Additional co-solvents which may be present are cosmetically acceptable organic solvents or a mixture of solvents with a boiling point below 400° C in an amount of from 0.1 to 15 % by weight, preferably from 1 to 10 % by weight. Particularly suitable additional co-solvents are unbranched or branched hydrocarbons, such as pentane, hexane, isopentane and cyclic hydrocarbons, such as cyclopentane and cyclohexane. Further, particularly preferred water-soluble solvents are glycerol, ethylene glycol and propylene glycol in an amount up to 30% by weight.

### Direct Dyes

Preferred among the direct hair dyes are the following compounds, alone or in combination with one another: Hydroxyethyl-2-nitro-p-toluidine, 2-hydroxy-ethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxy-ethyl)amino]-2-nitrobenzene hydrochloride (HC Blue No. 12), 1-amino-4-[di(2-hydroxy-ethyl)amino]-2-nitro-benzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-chloro-6-ethylamino-4-nitro-phenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-amino-phenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hy-droxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof.

Particularly preferred among the aforesaid direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxy-ethyl)amino]-2-nitrobenzene hydrochloride (HC Blue No. 12), 1-amino-4-[di(2-hydroxy-ethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)- amino]-2-nitrobenzene (HC Red No. 11), 2-chloro-6-ethylamino-4-nitro-phenol, 2-amino-6-chloro-4-nitrophenol, 4-[(2-hydroxyethyl)amino]-3-nitro-1-trifluoromethylben-zene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-amino-phenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hy-droxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof.

The total quantity of direct dyes in the composition of the invention amounts to about 0.01 to 15 % by weight, preferably 0.1 to 10 % by weight, most preferred 0.5 to 8 % by weight.

In one embodiment, the composition according to the invention comprises at least one viscosity-modifying substance in an amount of from preferably 0.01 to 20% by weight or 0.05 to 10% by weight or particularly preferably from 0.1 to 5% by weight.

The viscosity-modifying substance is preferably a thickening polymer, chosen from copolymers of at least one first monomer type, which is chosen from acrylic acid and methacrylic acid, and at least one second monomer type, which is chosen from esters of acrylic acid and ethoxylated fatty alcohol; crosslinked polyacrylic acid; crosslinked copolymers of at least one first monomer type, which is chosen from acrylic acid and methacrylic acid, and at least one second monomer type, which is chosen from esters of acrylic acid with C10- to C30-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated C10- to C30-alcohol and a third monomer type, chosen from C1- to C4-aminoalkyl acrylates; copolymers of two or more monomers chosen from acrylic acid, methacrylic acid, acrylic esters and methacrylic esters; copolymers of vinylpyrrolidone and ammonium acryloyldimethyltaurate; copolymers of ammonium acryloyldimethyltaurate and monomers chosen from esters of methacrylic acid and ethoxylated fatty alcohols; hydroxyethylcellulose; hydroxypropylcellulose; hydroxypropylguar; glyceryl polyacrylate; glyceryl polymethacrylate; copolymers of at least one C2-, C3- or C4-alkylene and styrene; polyurethanes; hydroxypropyl starch phosphate; polyacrylamide; copolymer of maleic anhydride and methyl vinyl ether crosslinked with decadiene; carob seed flour; guar gum; xanthan; dehydroxanthan; carrageenan; karaya gum; hydrolyzed corn starch; copolymers of polyethylene oxide, fatty alcohols and saturated methylenediphenyl diisocyanate (e.g. PEG-150/stearyl alcohol/SMDI copolymer).

### Surfactants:

In one embodiment, the composition according to the invention comprises 0.01 to 50% by weight, particularly preferably from 0.05 to 20% by weight, very particularly preferably from 0.1 to 15% by weight, of at least one emulsifier or surfactant. The surfactant may be nonionic with HLB > 8, anionic or zwitterionic.

### Nonionic Surfactants

From the nonionic surfactants optionally used those are preferred having an HLB (Hydrophilic Lipophilic Balance) of greater than 12. The nonionic surfactant of HLB >12 optionally present in the composition may be, for example, an ethoxylated or ethoxylated/propoxylated fatty alcohol with a fatty chain comprising from 12 to 22 carbon atoms, ethoxylated sterols, such as stearyl- or lauryl alcohol (EO-7); PEG-16 soya sterol or PEG-10 soya sterol, polyoxyethylene polyoxypropylene block polymers (poloxamers) and their mixtures. Ethoxylated sterols and of poloxamers are preferred.

Suitable nonionic surfactants are, for example,
- ethoxylated fatty alcohols, fatty acids, fatty acid glycerides or alkylphenols, in particular addition products of from 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group,
- C12- to C22-fatty acid mono- and diesters of addition products of from 1 to 30 mol of ethylene oxide onto glycerol,
- addition products of from 5 to 60 mol of ethylene oxide onto castor oil or onto hydrogenated castor oil,
- fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate,
- esters of sorbitan and one, two or three C8- to C22-fatty acids and a degree of ethoxylation of from 4 to 20,
- polyglyceryl fatty acid esters, in particular of one, two or more C8- to C22-fatty acids and polyglycerol having preferably 2 to 20 glyceryl units,
- alkyl glucosides, alkyl oligoglucosides and alkyl polyglucosides having C8 to C22-alkyl groups, e.g. decylglucoside or laurylglucoside.

The amount of nonionic surfactant in the emulsion and the cosmetic composition preferably can range from about 0.1 % to about 50 % by weight and more preferably from about 0.1 % to about 10 % by weight of the total weight of the emulsion.

### Anionic Surfactants

Suitable anionic surfactants are, for example, salts and esters of carboxylic acids, alkyl ether sulfates and alkyl sulfates, fatty alcohol ester sulfates, sulfonic acid and its salts (e.g. sulfosuccinates or fatty acid isethienates), phosphoric esters and their salts, acylamino acids and their salts. A detailed description of these anionic surfactants is to be found in the publication "FIEDLER - Lexikon der Hilfsstoffe [Lexikon of auxiliaries]", Volume 1, 5th Edition (2002), pages 97 to 102, to which reference is hereby expressly made. Preferred surfactants are mono-, di- and/or triesters of phosphoric acid whose addition products are from 2 to 30 mol of ethylene oxide onto C8- to C22-fatty alcohols.

### Amphoteric Surfactants

Suitable amphoteric surfactants are, for example, derivatives of aliphatic quaternary ammonium, phosphonium and sulfonium compounds of the formula 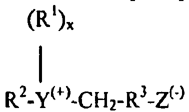 where R¹ is a straight-chain or branched-chain alkyl, alkenyl or hydroxyalkyl group having 8 to 18 carbon atoms and 0 to about 10 ethylene oxide units and 0 to 1 glycerol unit; Y is a N-, P- or S-containing group; R² is an alkyl or monohydroxyalkyl group having 1 to 3 carbon atoms; X is 1 if Y is a sulfur atom and X is 2 if Y is a nitrogen atom or a phosphorus atom; R³ is an alkylene or hydroxyalkylene group having 1 to 4 carbon atoms and Z⁽⁻⁾ is a carboxylate, sulfate, phosphonate or phosphate group.

Other amphoteric surfactants such as betaines are likewise suitable. Examples of betaines includes C8- to C18-alkylbetaines, such as cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)-alphacarboxyethylbetaine; C8- to C18-sulfobetaines, such as cocodimethyl-sulfopropylbetaine, stearyldimethylsulfopropylbetaine, lauryldimethyl-sulfoethylbetaine, laurylbis(2-hydroxyethyl)sulfopropylbetaine; the carboxyl derivatives of imidazole, the C8- to C18-alkyldimethylammonium acetates, the C8- to C18-alkyldimethylcarbonylmethylammonium salts, and the C8- to C18-fatty acid alkylamidobetaines, such as, for example, coconut fatty acid amidopropylbetaine and N-coconut fatty acid amidoethyl-N-[2-(carboxymethoxy)ethyl]glycerol (CTFA name: Cocoamphocarboxyglycinate).

### Pigments

In one preferred embodiment, the composition according to the invention comprises at least one pigment. These may be colored pigments which impart color effects to the product mass or to the hair, or they may be luster effect pigments which impart luster effects to the product mass or to the hair. The color or luster effects on the hair are preferably temporary, i.e. they last until the next hair wash and can be removed again by washing the hair with customary shampoos. The pigments are present in the product mass in undissolved form and may be present in an amount of from 0.01 to 25% by weight, particularly preferably from 5 to 15% by weight. The preferred particle size is 1 to 200 µm, in particular 3 to 150 µm, particularly preferably 10 to 100 µm. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin, for example produced from chalk, ocher, umber, green earth, burnt sienna or graphite. The pigments may be white pigments, such as, for example, titanium dioxide or zinc oxide, black pigments, such as, for example, iron oxide black, colored pigments, such as, for example, ultramarine or iron oxide red, luster pigments, metal effect pigments, pearlescent pigments, and fluorescent or phosphorescent pigments, where preferably at least one pigment is a colored, nonwhite pigment. Of suitability are metal oxides, hydroxides and oxide hydrates, mixed phase pigments, sulfur-containing silicates, metal sulfides, complex metal cyanides, metal sulfates, chromates and molybdates, and the metals themselves (bronze pigments). Of particular suitability are titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal).

Particular preference is given to pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine etc. and where the color can be determined by varying the layer thickness. Such pigments are sold, for example, under the trade names Rona®, Colorona®, Dichrona® and Timiron® by Merck, Germany.

Organic pigments are, for example, the natural pigments sepia, gamboge, bone charcoal, Cassel brown, indigo, chlorophyll and other plant pigments. Synthetic organic pigments are, for example, azo pigments, anthraquinoids, indigoids, dioxazine, quinacridone, phthalocyanine, isoindolinone, perylene and perinone, metal complex, alkali blue and diketopyrrolopyrrole pigments.

### Solid Particles

In another embodiment, the composition according to the invention comprises from 0.01 to 10% by weight, particularly preferably from 0.05 to 5% by weight, of at least one particulate substance. Suitable substances are, for example, substances which are solid at room temperature (25°C) and are in the form of particles. For example, silica, silicates, aluminates, clay earths, mica, insoluble salts, in particular insoluble inorganic metal salts, metal oxides, e.g. titanium dioxide, minerals and insoluble polymer particles are suitable.

The particles are present in the composition in undissolved, preferably stably dispersed form, and, following application to the hair and evaporation of the solvent, can deposit on the hair in solid form. A stable dispersion can be achieved by providing the composition A with a yield point which is large enough to prevent the solid particles from sinking. An adequate yield point can be established using suitable gel formers in a suitable amount.

Preferred particulate substances are silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate.

The additives are used in quantities usual for such purposes; for example, the wetting agents and emulsifiers in concentrations of a total of 0.2 % to 30 % by weight, the alcohols in a total quantity of 0.1% to 70 % by weight, the opacifiers, perfume oils and dyes in a quantity of 0.01 % to 1 % by weight each, the buffer substances in a total quantity of 0.1 % to 10 % by weight, and sugars, solubilizers, stabilizers, while the thickeners and solubilizers may be contained in this composition in a total quantity of 0.1 to 20 % by weight. Surfactants are preferably contained at levels of from about 0.1 % to about 5%, more preferably from about 0.1 % to about 1.5 %, most preferably from about 0.1 % to about 0.8 %, by weight of the composition.

In another preferred embodiment, the composition according to the invention comprises a cosmetically acceptable non aqueous volatile solvent. As used herein, the term "volatile" refers to liquids having a boiling point at one atmosphere of 260° C or less, preferably 250° C, more preferably 230° C or less, most preferably 225° C or less. In general, the boiling point of the volatile solvents will be at least about 50° C, preferably at least about 100° C.

The cosmetically acceptable volatile solvent is preferably a glycol ether of a mono-alcohol of 2 to 6 carbon atoms, more preferred an ethylene glycol or an propylene glycol ether of a mono-alcohol of 2 to 6 carbon atoms, more preferred of a mono-alcohol of 3 to 5 carbon atoms, most preferred it is ethylene glycol mono butyl ether.

The volatile solvent is preferably present in an amount of from 0.1 to 98 % by weight, more preferred in an amount of from 1 to 60 % by weight and most preferred in an amount of from 2 to 10 % by weight of the total weight of the hair treating composition.

### UV-Absobers

In one embodiment, the composition according to the invention comprises a photoprotective substance in an amount of from preferably 0.01 to 10% by weight or from 0.1 to 5% by weight, particularly preferably from 0.2 to 2% by weight. The photoprotective substances include, in particular, all of the photoprotective substances specified in EP 1 084 696. Preference is given to: 2-ethylhexyl 4-methoxycinnamate, methyl methoxycinnamate, 2-hydroxy-4-methoxybenzophenone-5-sulfonic acid and polyethoxylated p-aminobenzoates.

### Preservatives

In one embodiment, the composition according to the invention comprises 0.01 to 5% by weight, particularly preferably from 0.05 to 1% by weight, of at least one preservative. Suitable preservatives are the substances listed in the International Cosmetic Ingredient Dictionary and Handbook, 9th Edition with the function "preservatives", e.g. phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile and DMDM hydantoin.

### Viscosity

The hair shaping composition of the present invention preferably has a viscosity at 25°C of about 0.1 mPa · s to about 1,000,000 mPa · s, preferably from about 1 mPa · s to about 80,000 mPa · s, more preferably from about 5 mPa · s to about 3,500 mPa · s. Viscosity is determined - if not otherwise defined - by HAAKE Rotation Viscometer VT 550 with cooling/heating vessel and sensor systems according to DIN 53019 (MV-DIN, SV-DIN), shear rate is 12.9 s⁻¹.

### pH-Value

By varying the pH value, a composition can be made available that is universally suitable for every hair structure, optionally with the additional application of heat.

The hair treating composition preferably has a pH value of from 2.0 to 12.0, more preferred from 3.0 to 9.0 and most preferred from 4.5 to 7.5. As an alkalizing agent or agent for adjusting the pH value, ammonia or caustic soda is especially suitable, but water-soluble, physiologically tolerable salts of organic and inorganic bases can also be considered, such as ammonium hydrogen carbonate, ammonia, monoethanolamine, ammonium carbonate. Further suitable alkalizing agents may be selected from the group consisting of 2-amino-2-methyl-1-propanol, 2-amino-2-methyl-1,3-propanediol, 2-amino-2-ethyl-1,3-propanediol, tris(hydroxylmethyl)-aminomethane, 2-amino-1-butanole, tris-(2-hydroxypropyl)-amine, 2,2-iminobisethanol, lysine, iminourea (guanidine carbonate), tetrahydro-1,4-oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide and magnesium oxide.

To establish an acidic pH value, hydrochloric acid, phosphoric acid, acetic acid, formic acid, sulfuric acid, hydrochloric acid or citric acid can be used in particular. The acidic pH can be also adjusted with a buffer such as a phosphate buffer, a TRIS buffer or a citric buffer. The buffers may be used alone or in combination with an acid.

### Packaging

The composition may be sold in single- or dual- or triple-component packages.

In another embodiment, the composition according to the invention is in the form of a gel, in the form of a viscous lotion or in the form of a spray gel which is sprayed with a mechanical device and comprises at least one of the above mentioned thickening polymers in an amount of from preferably 0.05 to 10 % by weight, particularly preferably from 0.1 to 2 % by weight and has a viscosity of at least 250 mPa · s (measured using a Bohlin rheometer CS, measurement body C25 at 25°C and a shear rate of 50 s⁻¹). The viscosity of the gel is preferably from 500 to 50,000 mPa · s, particularly preferably from 1,000 to 15,000 mPa · s at 25°C.

In another embodiment, the composition according to the invention is in the form of an O/W emulsion, a W/O emulsion or a micro emulsion, and comprises at least one of the abovementioned oils or waxes emulsified in water, and at least one of the abovementioned surfactants.

In another embodiment, the composition according to the invention is in the form of a spray product, either in combination with a mechanical pump spray device or in combination with at least one propellant chosen from propane, butane, dimethyl ether and fluorinated hydrocarbons. If the composition is an aerosol spray it additionally comprises preferably 15 to 85 % by weight, particularly preferably 25 to 75 % by weight, of a propellant and is bottled in a pressurized container. Suitable propellants are, for example, lower alkanes, such as, for example, n-butane, isobutane and propane, or mixtures thereof, and dimethyl ether or fluorinated hydrocarbons, such as F 152a (1,1-difluoroethane) or F 134 (tetrafluoroethane), and also propellants which are in gaseous form at the pressures under consideration, such as, for example, N₂, N₂O and CO₂, and mixtures of the abovementioned propellants.

If the composition of the invention is formulated as a non-aerosol hairspray it is sprayed out from the container with the help of a suitable mechanically operated spraying device. Mechanical spraying devices are understood as meaning those devices which permit the spraying of a composition without use of a propellant. A suitable mechanical spray device used may, for example, be a spray pump or an elastic container provided with a spray valve in which the cosmetic composition according to the invention is bottled under pressure, where the elastic container expands and from which the composition is continuously dispensed as a result of the elastic container contracting upon opening the spray valve.

In another embodiment, the composition according to the invention is in the form of a foamable product (mousse) in combination with a devices for foaming, comprises at least one customary foam-imparting substance known for this purpose, e. g. at least one foam-forming surfactant or at least one foam-forming polymer. Devices for foaming are understood as meaning those devices which enable the foaming of a liquid with or without use of a propellant. A suitable mechanical foaming device which can be used is, for example, a standard commercial pump foamer or an aerosol foaming head. The product is either in combination with a mechanical pump foaming device (pump foam) or in combination with at least one propellant (aerosol foam) in an amount of from preferably 1 to 20% by weight, in particular from 2 to 10 % by weight. Propellants are chosen, for example, from propane, butane, dimethyl ether and fluorinated hydrocarbons. The composition is foamed directly prior to application and incorporated into the hair as foam and can then be rinsed out or left in the hair without rinsing.

The foamable product comprise, as active ingredients or additives, preferably polymers which are chosen from chitosan, chitosan salts, chitosan derivatives, cationic cellulose compounds, copolymers of vinylpyrrolidone, vinylcaprolactam and a basic acrylamide monomer or mixtures of these polymers. Suitable chitosan salts, chitosan derivatives, cationic cellulose derivatives are, for example, those mentioned above. Preferred cationic cellulose compounds are copolymers of hydroxyethylcellulose and diallyldimethylammonium chloride (polyquaternium-4) and reaction products of hydroxyethylcellulose and epoxides substituted by a trialkylammonium group (Polyquaternium-10). Preferred chitosan salts are the salts with formic acid, lactic acid and pyrrolidonecarboxylic acid. Preferred copolymers of vinylpyrrolidone, vinylcaprolactam and a basic acrylamide monomer are those in which the acrylamide monomer is dimethylaminopropylacrylamide. Also preferred are foamable products which comprise copolymers of hydroxyethylcellulose and diallyldimethylammonium chloride (polyquaternium-4) and copolymers of vinylpyrrolidone, vinylcaprolactam and dimethylaminopropylacrylamide, and foamable products which comprise copolymers of hydroxyethylcellulose and diallyldimethylammonium chloride (polyquaternium-4) and copolymers of vinylpyrrolidone, vinylcaprolactam and dimethylaminopropylacrylamide, and at least one chitosan salt.

In another embodiment, the composition according to the invention is in the form of a hair wax, i.e. it has a wax-like consistency and comprises at least one of the abovementioned waxes in an amount of from preferably 0.5 to 30 % by weight, and optionally further water-insoluble substances. The wax-like consistency is preferably characterized in that the needle penetration number (unit of measurement 0.1 mm, test weight 100 g, test time 5 s, test temperature 25°C; according to DIN 51 579) is greater than or equal to 10, particularly preferably greater than or equal to 20 and that the solidification point of the product is preferably greater than or equal to 30°C and less than or equal to 70°C, particularly preferably in the range from 40 to 55°C. Suitable waxes and water-insoluble substances are, in particular, emulsifiers with an HLB value below 7, silicone oils, silicone waxes, waxes (e.g. wax alcohols, wax acids, wax esters, and in particular natural waxes such as beeswax, carnauba wax, etc.), fatty alcohols, fatty acids, fatty acid esters or hydrophilic waxes, such as, for example, high molecular weight polyethylene glycols with a molecular weight of from 800 to 20,000 g/mol, preferably from 2,000 to 10,000 g/mol.

If the hair-treatment composition according to the invention is in the form of a hair lotion, then it has the form of an essentially non-viscous or low-viscosity, flowable solution, dispersion or emulsion with a content of at least 10% by weight, preferably 20 to 95% by weight, of a cosmetically compatible alcohol. Alcohols which can be used are, in particular, the lower alcohols having 1 to 4 carbon atoms customarily used for cosmetic purposes, e.g. ethanol and isopropanol.

If the hair-treatment composition according to the invention is in the form of a hair cream, then it is preferably in the form of an emulsion and comprises either additionally viscosity-imparting ingredients in an amount of from 0.1 to 10 % by weight, or the required viscosity and creamy consistency is built up in a customary manner through micelle formation with the help of suitable emulsifiers, fatty acids, fatty alcohols, waxes etc.

In another aspect of the invention there is provided a method for reducing the time to dry wet hair, for example, drying wet hair following showering, shampooing, bathing, swimming, and the like, or to shorten the drying time of an aqueous hair treating composition comprising applying to dry or wet hair the above specified hair treating composition containing the polydimethylsiloxane (P) as recited in claim 11 and subsequently drying the hair.

Also disclosed is a method to shorten the drying time of an aqueous hair treating composition on the head of a person and simultaneously to protect the hair dye on said hair from being washed out is provided, comprising the steps of:
1. (a) applying a hair dying composition onto the hair to be dyed on the head of a person,
2. (b) leaving the hair dying composition on the hair for a time required to dye said hair,
3. (c) optionally rinsing said hair with water or an aqueous acidic composition,
4. (d) optionally towelling and/or drying said hair,
5. (e) applying to said hair the above specified polydimethylsiloxane (P) and the protonating agent or the above specified hair treating composition containing the polydimethylsiloxane (P) and the protonating agent and
6. (f) subsequently drying said hair.

In a further preferred embodiment it is provided a method for dying the hair on the head of a person while simultaneously reducing the time to dry said hair and to protect the hair dye on said hair from being washed out, as recited in claim 21 comprising the steps of:
1. (a) applying a hair dying composition onto the hair to be dyed,
2. (b) leaving the hair dying composition on the hair for a time required to dye said hair,
3. (c) optionally rinsing said hair with water or an aqueous acidic composition,
4. (d) optionally towelling and/or drying said hair,
5. (e) applying to said hair the above specified polydimethylsiloxane (P) and the protonating agent or the above specified hair treating composition containing the polydimethylsiloxane (P) and the protonating agent as recited in claim 21 and
6. (f) subsequently drying said hair.

Before or during the drying step, the hair may be mechanically processed to help water to sheet from the hair. Such processing includes brushing, combing, towelling, and the like. In the case of towelling, it is seen that mechanical action is typically combined with absorbing of the water present on the hair. In the case of blow drying of the hair, it is seen that the force of the hot air from the dryer will also cause the water to sheet from the hair. Surprisingly, the time to dry the hair is reduced, notwithstanding the application to hair of additional water.

In a further preferred embodiment of the invention a method for enhancing the durability of a hair shape against humidity, water and hair washings while simultaneously reducing the time to dry said hair is provided, comprising the steps of:
1. (a) shaping the hair,
2. (b) optionally rinsing said hair with water or an aqueous acidic composition,
3. (c) optionally towelling and/or drying said hair,
4. (d) applying to said hair the above specified polydimethylsiloxane (P) and the protonating agent or the above specified hair treating composition containing the polydimethylsiloxane (P) and the protonating agent as recited in claim 11 and
5. (e) subsequently drying said hair.

In a further preferred embodiment a method for enhancing the durability of a hair shape against humidity, water and hair washings while simultaneously reducing the time to dry said hair is provided, comprising the steps of:
1. (a) washing said hair,
2. (b) optionally towelling and/or drying said hair,
3. (c) applying to said hair the above specified polydimethylsiloxane (P) and the protonating agent or the above specified hair treating composition containing the polydimethylsiloxane (P) and the protonating agent as recited in claim 11 and
4. (d) subsequently drying and simultaneously shaping said hair.

The examples below serve to illustrate the subject matter of the invention in more detail. Unless stated otherwise, the polymer contents given in each case refer to the solids content.

### EXAMPLES

### Substances used in the examples:

### Amino-Functional Organopolysiloxanes Used:

### Amine Oil 1:

Amine Oil 1 has a viscosity of about 1000 mm²/s at 25° C., the functional radicals are -CH₂)₃NH(CH₂)₂NH₂ and has an amine number of 0.6 meq/g of organopolysiloxane. In addition, the organopolysiloxane contains about 0.75 mol % of reactive OMe/OH radicals as terminal groups.

### Amine Oil 2:

Amine Oil 2 has a viscosity of about 1000 mm²/s at 25° C, the functional radicals are -CH₂)₃NH(CH₂)₂NH₂ and has an amine number of 0.6 meq/g of organopolysiloxane. The terminal groups in this case are Me₃SiO radicals.

### Amine Oil 3:

Amine oil 3 has a viscosity of about 230 mm²/s at 25° C., the functional radicals are -(CH₂)₃NH(CH₂)₂NH₂, and an amine number of 2.6 meq/g of organopolysiloxane. The terminal groups are likewise Me₃SiO radicals.

### Emulsions

### Emulsion A

16.g of Amine Oil 1 are added to 6 g of water, 6 g of ethylene glycol monobutyl ether and 0.17 g of acetic acid at room temperature while stirring, and the remaining water to a total weight of 100 g is then also incorporated. A milky opaque emulsion is obtained. The emulsion is added at room temperature as last ingredient to the active solution/dispersion of the following examples.

### Emulsion B

16.g of a mixture of 11.1 g of Amine Oil 2 and 4.9 g of MQ resin are added to 6 g of water, 6 g of ethylene glycol monobutyl ether and 0.17 g of acetic acid at room temperature while stirring, and the remaining water to a total weight of 100 g is then also incorporated. A milky opaque emulsion is obtained. The emulsion is added at room temperature as last ingredient to the active solution/dispersion of the following examples.

### Emulsion C

16.g of a mixture of 11.1 g of Amine Oil 3 and 4.9 g of MQ resin are added to 6 g of water and 0.17 g of acetic acid at room temperature while stirring, and the remaining water to a total weight of 100 g is then also incorporated. A milky opaque emulsion is obtained. The emulsion is added at room temperature as last ingredient to the active solution/dispersion of the following examples.

### Trade names used in the examples

| | |
|---|---|
| Abilquat® 3270: | Quaternium-80, 50% in propylene glycol (Goldschmidt) |
| Aculyn® 48: | PEG-150/STEARYL ALCOHOL/SMDI COPOLYMER, 19% in water (Rohm and Haas) |
| AMP 95% | Aminomethylpropanol, 95% aqueous solution |
| Amphomer® | OCTYLACRYLAMIDE/ACRYLATES/BUTYL-AMINOETHYLMETHACRYLATE COPOLYMER |
| Aristoflex® AVC | AMMONIUM ACRYLOYLDIMETHYL-TAURATE/VP COPOLYMER |
| Aquaflex® FX-64: | ISOBUTYLENE/ETHYLMALEIMIDE/- HYDROXYETHYLMALEIMIDE COPOLYMER, 40% strength in water/ethanol (ISP) |
| Aquaflex® SF 40: | VP/VINYL CAPROLACTAM/DMAPA ACRYLATES COPOLYMER, 40% in ethanol (ISP) |
| Advantage® S | VINYL CAPROLACTAM/VP/DIMETHYL-AMINOETHYL METHACRYLATE COPOLYMER |
| Carbomer (Carbopol) | Acrylic acid homopolmer |
| Celquat® L200: | Copolymer of hydroxyethylcellulose and diallyldimethylammonium chloride; Polyquaternium-4 |
| GENAMIN CTAC 50 | CTFA: Cetrimonium Chloride; Cetyltrimethylammonium chloride |
| Copolymer 845: | VP/DIMETHYLAMINOETHYLMETHACRYL-ATE COPOLYMER, 20% in water (ISP) |
| Dehydol® LS 4 | Lauryl alcohol tetraoxyethylen ether |
| Dekaben® LMB: | IODOPROPYNYL BUTYLCARBAMATE, 10% strength in butylene glycol |
| Dekaben® LMP: | Phenoxyethanol and iodopropynyl butylcarbamate |
| Diaformer Z-711: | ACRYLATES/LAURYL ACRYLATE/STEARYL ACRYLATE/ETHYLAMINE OXIDE METHACRYLATE COPOLYMER, 40% (Clariant) |
| Dow Corning 1401: | High molecular weight Dimethiconol, 13% in cyclomethicone |
| Eumulgin® L: | INCI: PEG-1-PEG-9 LAURYL GLYCOL ETHER |
| Flexan® | Sodium polystyrenesulfonate |
| GAFQUAT® 755 N | CTFA: Polyquaternium-11 |
| Jaguar C-17/162 | CTFA: GUAR HYDROXYPROPYLTRIMONIUM CHLORIDE |
| Laureth-4 | Lauryl alcohol tetraoxyethylen ether |
| Luviset® Clear: | Terpolymer of vinylpyrrolidone, methacrylamide and vinylimidazole (BASF) |
| Luviskol® VA 64 | Vinylpyrrolidone/vinylacetate copolymer |
| Luviskol® K 90 Powder | Vinylpyrrolidone |
| Luvimer® 100 P | t-butyl acrylate/ethyl acrylate/methacrylic acid copolymer |
| Natrosol® G: | Hydroxyethylcellulose |
| Pemulen®: | ACRYLATES/C10-30 ALKYL ACRYLATE CROSSPOLYMER |
| Structure® 3001: | ACRYLATES/CETETH-20 ITACONATE COPOLYMER 30% strength in water (National Starch) |
| Surfactant 193: | Ethoxylated dimethylpolysiloxane; INCI: PEG-12 Dimethicone (Dow Corning) |
| Synthalen® W 2000: | ACRYLATES/PALMETH-25 ACRYLATE COPOLYMER (31 % in water) |
| Tego Betain L 5045 | CTFA: COCAMIDOPROPYL BETAINE |

### Example 1: Liquid gel

| | |
|---|---|
| Emulsion A | 2.00 g |
| Luviset® Clear | 1.00 g |
| Surfactant 193 | 1.50 g |
| Carbomer | 0.30 g |
| AMP 95% | 0.30 g |
| Emulgin L | 0.20 g |
| Perfume | 0.15 g |
| Natrosol® G | 0.40 g |
| Ethanol | 16.50 g |
| Water | ad 100.00 g |

### Example 2: Liquid gel

| | |
|---|---|
| Emulsion B | 3.00 g |
| Luviset® Clear | 1.00 g |
| Vinylpyrrolidone/vinyl acetate copolymer | 1.00 g |
| Direct dye | 0.20 g |
| Surfactant 193 | 1.00 g |
| Xanthan Gum | 1.20 g |
| Citric acid | 0.10 g |
| Perfume | 0.15 g |
| Ethanol | 6.50 g |
| DMDM Hydantoin | 0.30 g |
| Water | ad 100.00 g |

### Example 3: Liquid gel

| | |
|---|---|
| Emulsion B | 4.00 g |
| Luviset® Clear | 0.50 g |
| Glucose | 7.00 g |
| Direct dye | 0.50 g |
| Propylene glycol | 3.80 g |
| Hydroxypropylguar | 0.30 g |
| AMP 95% | 0.20 g |
| PEG-25 PABA | 0.50 g |
| PEG-40 Hydrogenated Castor Oil | 0.18 g |
| PPG-1-PEG-9 Lauryl Glycol Ether | 0.18 g |
| Perfume | 0.15 g |
| Ethanol | 16.50 g |
| Water | ad 100.00 g |

### Example 4: Spray gel

| | |
|---|---|
| Emulsion C | 1.00 g |
| Luviset® Clear | 1.50 g |
| Luviskol® VA 64 | 3.00 g |
| Ethanol | 18.00 g |
| Aminomethylpropanol 95% aqueous solution | 0.10 g |
| PEG-40 Hydrogenated Castor Oil | 0.20 g |
| Perfume | 0.20 g |
| Aculyn® 48 | 0.50 g |
| Water | ad 70.00 g |

The composition is packaged in a packaging with pump spray device.

### Example 5: Rapidly Drying Gel

| | |
|---|---|
| Emulsion A | 1.00 g |
| Luviset® Clear | 3.00 g |
| Aquaflex® SF 40 | 2.80 g |
| Surfactant 193 | 1.50 g |
| Pemulen® | 0.35 g |
| AMP 95% | 0.26 g |
| Methylmethoxycinnamate | 0.30 g |
| Perfume | 0.30 g |
| Ethanol | 34.20 g |
| Water | ad 100.00 g |

### Example 6: Rapidly Drying Gel Spray

| | |
|---|---|
| Emulsion A | 0.80 g |
| Luviset® Clear | 1.00 g |
| Surfactant 193 | 1.00 g |
| Carbomer (Carbopol) | 0.23 g |
| AMP 95% | 0.22 g |
| Perfume | 0.15 g |
| Ethanol | 6.50 g |
| Water | ad 100.00 g |

The composition is packaged in a packaging with pump spray device.

### Example 7: Blow-drying gel

| | |
|---|---|
| Emulsion A | 1.80 g |
| Luviset® Clear | 1.00 g |
| Surfactant 193 | 1.00 g |
| Hydroxypropylcellulose (Klucel® HF) | 0.95 g |
| Citric acid | 0.10 g |
| Perfume | 0.15 g |
| Ethanol | 6.50 g |
| Water | ad 100.00 g |

### Example 8: Rapid Drying Gel

| | |
|---|---|
| Emulsion B | 2.90 g |
| Luviset® Clear | 1.00 g |
| Polyvinylpyrrolidone K 90 | 1.80 g |
| Direct dye | 1.00 g |
| Surfactant 193 | 1.50 g |
| Synthalen® W 2000 | 1.00 g |
| AMP 95% | 0.30 g |
| PEG-25 PABA (Uvinul® P 25) | 0.30 g |
| Panthenol | 0.15 g |
| Perfume | 0.30 g |
| Ethanol | 34.20 g |
| Keratin hydrolysate | 0.10 g |
| Water | ad 100.00 g |

### Example 9: Gel - strong hold

| | |
|---|---|
| Emulsion A | 3.80 g |
| Luviset® Clear | 1.00 g |
| VA/CROTONATES COPOLYMER (Luviset® CA 66) | 2.50 g |
| Sorbitol | 4.20 g |
| Direct dye | 0.?? g |
| Carbomer (Tego Carbomer) | 0.80 g |
| AMP 95% | 0.30 g |
| Methylparaben | 0.20 g |
| PEG-40 Hydrogenated Castor Oil | 0.20 g |
| Panthenol | 0.10 g |
| Perfume | 0.20 g |
| Ethanol | 5.00 g |
| Water | ad 100.00 g |

### Example 10: Gel - strong hold

| | |
|---|---|
| Emulsion A | 5.80 g |
| Luviset® Clear | 1.00 g |
| Aquaflex® SF 40 | 1.50 g |
| Vinyl acetate/crotonic acid copolymer | 1.20 g |
| Sorbitol | 4.20 g |
| Structure® 3001 | 0.12 g |
| AMP 95% | 0.35 g |
| PEG-25 PABA | 0.50 g |
| Dekaben® LMB | 0.20 g |
| PEG-40 Hydrogenated Castor Oil | 0.20 g |
| Panthenol | 0.10 g |
| Perfume | 0.20 g |
| Ethanol | 5.00 g |
| Water | ad 100.00 g |

### Example 11: Gel - normal hold

| | |
|---|---|
| Emulsion B | 3.80 g |
| Luviset® Clear | 1.50 g |
| Glycerol | 5.20 g |
| Propylene glycol | 4.00 g |
| Ammonium Acryloyldimethyltaurate/VP Copolymer (Aristoflex® AVC) | 0.35 g |
| AMP 95% | 0.26 g |
| Polysorbate-40 | 1.00 g |
| Methylparaben | 0.20 g |
| PEG-25 PABA | 0.50 g |
| Perfume | 0.20 g |
| Ethanol | 4.50 g |
| Water | ad 100.0 g |

### Example 12: Pump - setting foam

| | |
|---|---|
| Emulsion A | 0.20 g |
| Luviset® Clear | 1.30 g |
| Vinyl acetate/crotonic acid copolymer | 0.30 g |
| Cocamidopropyl Hydroxysultaine | 0.40 g |
| Citric acid | 0.10 g |
| Ethanol | 8.90 g |
| Betaine | 0.10 g |
| Perfume | 0.15 g |
| Water | ad 100.00 g |

The composition is packaged in a packaging with mechanically operated pump foaming device.

### Example 13: Pump - setting foam

| | |
|---|---|
| Emulsion A | 1.20 g |
| Luviset® Clear | 1.50 g |
| Acrylic acid/ethyl acrylate/N-tert-butylacrylamide Copolymer | 0.40 g |
| Direct dye | 1.90 g |
| Cocamidopropyl Hydroxysultaine | 0.40 g |
| Citric acid | 0.10 g |
| Dekaben® LMP | 0.20 g |
| Camomile blossom extract | 0.10 g |
| Perfume | 0.15 g |
| Water | ad 100.00 g |

The composition is packaged in a packaging with mechanically operated pump foaming device.

### Example 14: Pump - setting foam

| | |
|---|---|
| Emulsion A | 7.20 g |
| Luviset® Clear | 1.20 g |
| Polyquaternium-6 | 0.35 g |
| Cocamidopropyl Hydroxysultaine | 0.40 g |
| Panthenol | 0.10 g |
| Ethanol | 8.90 g |
| Betaine | 0.10 g |
| Perfume | 0.15 g |
| Water | ad 100.00 g |

The composition is packaged in a packaging with mechanically operated pump foaming device.

### Example 15: Pump - setting foam

| | |
|---|---|
| Emulsion A | 1.20 g |
| Luviset® Clear | 2.50 g |
| Direct dye | 3.00 g |
| Ethanol | 8.90 g |
| Cocamidopropyl Hydroxysultaine | 0.20 g |
| Cetyltrimethylammonium chloride | 0.20 g |
| Perfume | 0.15 g |
| Silk fibroin hydrolysate (Silkpro®) | 0.10 g |
| Water | ad 100.00 g |

The composition is packaged in a packaging with mechanically operated pump foaming device.

### Example 16: Pump - setting foam

| | |
|---|---|
| Emulsion A | 2.20 g |
| Luviset® Clear | 2.00 g |
| Celquat® L200 | 0.30 g |
| Direct dye | 0.80 g |
| Ethanol | 8.90 g |
| Cocamidopropyl Hydroxysultaine | 0.20 g |
| Cetyltrimethylammonium chloride | 0.20 g |
| Perfume | 0.15 g |
| Citric acid | 0.10 g |
| Betaine | 0.10 g |
| Water | ad 100.00 g |

The composition is packaged in a packaging with mechanically operated pump foaming device.

### Example 17: Pump - setting foam

| | |
|---|---|
| Emulsion B | 1.20 g |
| Luviset® Clear | 1.30 g |
| Polyquaterium-11 | 0.30 g |
| Direct dye | 0.20 g |
| Cocamidopropyl Hydroxysultaine | 0.40 g |
| Propylene glycol | 1.00 g |
| Methylparaben | 0.20 g |
| Perfume | 0.15 g |
| Water | ad 100.00 g |

The composition is packaged in a packaging with mechanically operated pump foaming device.

### Example 18: Pump - setting foam

| | |
|---|---|
| Emulsion C | 1.20 g |
| Luviset® Clear | 1.80 g |
| Direct dye | 1.90 g |
| Cocamidopropyl Hydroxysultaine | 0.40 g |
| Rosemary leaf extract (Extrapon® Rosemary) | 0.10 g |
| Ethanol | 8.90 g |
| Extrapon® seven herbs - extract | 0.10 g |
| Panthenyl ethyl ether | 0.10 g |
| Perfume | 0.15 g |
| Water | ad 100.00 g |

The composition is packaged in a packaging with mechanically operated pump foaming device.

### Example 19: Aerosol - setting foam - normal hold

| | |
|---|---|
| Emulsion A | 4.20 g |
| Luviset® Clear | 1.50 g |
| Butyl monoester of methyl vinyl ether/maleic acid copolymer | 0.50 g |
| Butane | 4.00 g |
| Propane | 4.00 g |
| Ethanol | 8.90 g |
| PEG-25 PABA | 0.40 g |
| Betaine | 0.15 g |
| Perfume | 0.15 g |
| Laureth-4 | 0.20 g |
| Cetrimonium bromide | 0.05 g |
| Amodimethicone | 0.50 g |
| Water | ad 100.00 g |

The composition is bottled in an aerosol can with foaming head.

### Example 20: Aerosol - setting foam - normal hold

| | |
|---|---|
| Emulsion A | 5.20 g |
| Luviset® Clear | 1.50 g |
| Polyquaternium-47 | 0.50 g |
| Butane | 4.00 g |
| Propane | 4.00 g |
| Betaine | 0.15 g |
| Dow Corning 1401 | 0.25 g |
| 2-Ethylhexyl 4-methoxycinnamate | 0.20 g |
| Perfume | 0.15 g |
| Laureth-4 | 0.20 g |
| Cetrimonium chloride | 0.07 g |
| Water | ad 100.00 g |

The composition is bottled in an aerosol can with foaming head.

### Example 21: Aerosol - setting foam - extra strong hold

| | |
|---|---|
| Emulsion A | 3.20 g |
| Luviset® Clear | 2.10 g |
| Copolymer 845 | 2.50 g |
| Polyquaternium-4 | 1.00 g |
| Butane | 4.00 g |
| Propane | 4.00 g |
| Panthenol | 0.20 g |
| Perfume | 0.20 g |
| Abilquat® 3270 | 0.70 g |
| Cetrimonium chloride | 0.07 g |
| Water | ad 100.00 g |

The composition is bottled in an aerosol can with foaming head.

### Example 22: Aerosol - setting foam - extra strong hold

| | |
|---|---|
| Emulsion A | 1.20 g |
| Luviset® Clear | 2.10 g |
| Vinyl acetate/crotonic acid copolymer | 0.60 g |
| Polyquaternium-7 | 0.50 g |
| Butane | 4.00 g |
| Propane | 4.00 g |
| Ethanol 510 | 8.90 g |
| PEG-25 PABA | 0.40 g |
| Panthenol | 0.20 g |
| Perfume | 0.20 g |
| Laureth-4 | 0.20 g |
| C9-C 11 Pareth-8 | 0.07 g |
| Water | ad 100.00 g |

The composition is bottled in an aerosol can with foaming head.

### Example 23: Setting spray

| | |
|---|---|
| Emulsion A | 0.20 g |
| Luviset® Clear | 1.50 g |
| Aquaflex® FX-64 | 1.00 g |
| Ethanol | 2.70 g |
| Polyquaternium-35 | 1.00 g |
| PEG-25 PABA | 0.70 g |
| Panthenol | 0.35 g |
| Perfume | 0.25 g |
| Cetrimonium chloride | 0.20 g |
| PEG-40 Hydrogenated Castor Oil | 0.21 g |
| Water | ad 100.00 g |

The composition is bottled in a packaging with pump spray device.

### Example 24: Setting spray

| | |
|---|---|
| Emulsion B | 1.20 g |
| Luviset® Clear | 2.50 g |
| Octylacrylamide/Acrylates/Butylaminoethylmethacrylate Copolymer (Amphomer®) | 2.00 g |
| Ethanol | 28.50 g |
| Aminomethylpropanol 95% | 0.60 g |
| Perfume | 0.25 g |
| Cetyltrimethylammonium bromide | 0.20 g |
| Water | ad 60.00 g |

The composition is bottled in a packaging with pump spray device.

### Example 25: Setting spray

| | |
|---|---|
| Emulsion B | 2.20 g |
| Luviset® Clear | 1.00 g |
| Octylacrylamide/Acrylates/Butylaminoethylmethacrylate Copolymer (Amphomer®) | 0.65 g |
| Celquat® L200 | 0.20 g |
| Ethanol | 28.5 g |
| Aminomethylpropanol 95% | 0.60 g |
| Perfume | 0.25 g |
| Cetyltrimethylammonium chloride | 0.20 g |
| Water | ad 60.00 g |

The composition is bottled in a packaging with pump spray device.

### Example 26: Non-aerosol blow-drying Lotion

| | |
|---|---|
| Emulsion C | 3.50 g |
| Luviset® Clear | 2.80 g |
| Vinyl Caprolactam/VP/Dimethylaminoethyl Methacrylate Copolymer (Advantage® S) | 2.00 g |
| Ethanol | 28.50 g |
| Perfume | 0.25 g |
| Cetyltrimethylammonium chloride | 0.20 g |
| Water | ad 60.00 g |

### Example 27: Nonaerosol blow-drying lotion

| | |
|---|---|
| Emulsion C | 1.50 g |
| Luviset® Clear | 3.10 g |
| Celquat® L200 | 0.05 g |
| Diaformer® Z-711 | 0.50 g |
| Ethanol | 27.00 g |
| Betaine | 0.10 g |
| Perfume | 0.25 g |
| PEG-40 Hydrogenated Castor Oil | 0.21 g |
| Cetyltrimethylammonium bromide | 0.20 g |
| Water | ad 100.00 g |

### Example 28: Nonaerosol blow-drying Lotion

| | |
|---|---|
| Emulsion C | 2.50 g |
| Luviset® Clear | 3.00 g |
| Sodium polystyrenesulfonate (Flexan®) | 2.30 g |
| Perfume | 0.20 g |
| Phenyltrimethicone (Baysilon® oil PD 5) | 0.02 g |
| Water | 10.00 g |
| Ethanol | ad 100.00 g |

The active ingredient solution is bottled in the ratio 45:55 with DME as propellant in an aerosol can.

### Example 29: VOC 80 Pump Spray - strong hold

| | |
|---|---|
| Emulsion A | 1.50 g |
| Luviset® Clear | 6.50 g |
| t-Butyl acrylate/Ethyl acrylate/Methacrylic acid Copolymer (Luvimer® 100 P) | 0.50 g |
| Perfume | 0.20 g |
| AMP | 0.10 g |
| Betaine | 0.05 g |
| Ethanol | 55.00 g |
| Demineralized water | ad 100.00 g |

The composition is bottled in a packaging with pump spray device.

### Example 30: Aerosol - hairspray

| | |
|---|---|
| Emulsion A | 2.80 g |
| Octylacrylamide/Acrylic acid/Butylaminoethyl methacrylate/Methyl methacrylate/hydroxypropyl methacrylate Copolymer (Amphomer®) | 3.00 g |
| Luviset® Clear | 1.50 g |
| Phenyl trimethicone (Baysilon® oil PD 5) | 0.02 g |
| Perfume | 0.20 g |
| Water | 10.00 g |
| AMP 95% | 0.48 g |
| Ethanol 510 | ad 100.00 g |

The active ingredient solution is bottled in the ratio 45:55 with DME as propellant in an aerosol can.

### Example 31: Aerosol - hairspray

| | |
|---|---|
| Emulsion A | 1.90 g |
| t-Butyl acrylate/Ethyl acrylate/Methacrylic acid Copolymer (Luvimer® 100 P) | 3.30 g |
| Luviset® Clear | 3.30 g |
| VA/CROTONATES COPOLYMER (Luviset® CA 66) | 1.00 g |
| Perfume | 0.20 g |
| Water | 10.00 g |
| AMP 95% | 0.84 g |
| Ethanol | ad 100.00 g |

The active ingredient solution is bottled in the ratio 45:55 with DME as propellant in an aerosol can.

### Example 32: Aerosol - hairspray

| | |
|---|---|
| Emulsion A | 4.20 g |
| Luviset® Clear | 2.50 g |
| t-Butyl acrylate/Ethyl acrylate/Methacrylic acid Copolymer (Luvimer® 100 P) | 3.30 g |
| Aminomethylpropanol 95% | 0.85 g |
| Perfume | 0.20 g |
| Baysilon® oil PD 5 | 0.02 g |
| Water | 10.00 g |
| Ethanol | ad 100.00 g |

The active ingredient solution is bottled in the ratio 45:55 with DME as propellant in an aerosol can.

### Example 33: Volumizing aerosol foam

| | |
|---|---|
| Emulsion B | 7.20 g |
| Luviset® Clear | 1.90 g |
| Celquat® L200 | 0.90 g |
| Aquaflex® SF 40 | 0.40 g |
| Laureth-4 | 0.20 g |
| Cetrimonium chloride | 0.10 g |
| Perfume | 0.10 g |
| Butane | 2.20 g |
| Propane | 3.00 g |
| Isobutane | 0.80 g |
| Water | ad 100.00 g |

The composition is bottled in an aerosol can with foaming head. Through use of the product on the hair, the hairstyle is given long-lasting volume.

### Example 34: Volumizing aerosol foam

| | |
|---|---|
| Emulsion B | 8.00 g |
| Luviset® Clear | 1.10 g |
| Chitosan | 1.00 g |
| Celquat® L200 | 0.90 g |
| Aquaflex® SF 40 | 0.40 g |
| Pyrrolidone carboxylic acid | 0.85 g |
| Laureth-4 | 0.20 g |
| Cetrimonium chloride | 0.10 g |
| Perfume | 0.10 g |
| Butane | 2.20 g |
| Propane | 3.00 g |
| Isobutane | 0.80 g |
| Water | ad 100.00 g |

The composition is bottled in an aerosol can with foaming head. Through use of the product on the hair, the hairstyle is given long-lasting volume.

### Example 35: Volumizing aerosol foam

| | |
|---|---|
| Emulsion A | 1.20 g |
| Luviset® Clear | 2.00 g |
| Chitosan | 0.27 g |
| Celquat® L200 | 1.00 g |
| Pyrrolidone carboxylic acid | 0.23 g |
| Direct dye | 0.90g |
| Laureth-4 | 0.20 g |
| Cetrimonium chloride | 0.10 g |
| Perfume, preservative | 0.50 g |
| Water | ad 100.00 g |

The composition is bottled with propane/butane 4.8 bar in the ratio of active ingredient solution : propellant gas = 94 : 6 in an aerosol can with foaming head. Through use of the product on the hair, the hairstyle is given long-lasting volume.

### Example 36: Rinse out Conditioner not within the scope of the present invention

| | |
|---|---|
| Emulsion A | 3.00 g |
| cetyltrimethyl ammonium chloride | 1.00 g |
| polymethylphenyl siloxane (CTFA: OUATERNIUM-80; Abil Quat© 3272) | 1.00 g |
| phenoxy ethanol | 0.40 g |
| PHB-methylester | 0.20 g |
| Copolymer of aminoethyl aminopropyl siloxane and dimethyl siloxane emulsion as a mixture with polyethylenglycol ether of tridecyl alcohol and cetyl trimethyl ammoniumchloride (CTFA: AMODIMETHICONE & TRIDECETH-12 & CETRIMONIUM CHLORIDE; Dow Corning 949 Cationic Emulsion®) | 1.00 g |
| isododecane | 5.00 g |
| perfume oil | 0.40 g |
| Water | ad 100.00 g |

### Example 37: Leave in Conditioner not within the scope of the present invention

| | |
|---|---|
| Emulsion B | 1.00 g |
| 2-hydroxy-3-(trimethylamonio)propylether chloride guar gum | 0.50 g |
| sodium benzoate | 0.50 g |
| glyoxylic acid | 0.10 g |
| creatine | 0.20 g |
| behenyl trimethylammonium chloride | 0.80 g |
| cetylstearyl alcohol | 0.60 g |
| stearic acid polyethylenglycol (20 EO) | 0.10 g |
| hydrolyzed silk | 0.10 g |
| perfume oil | 0.20 g |
| water | ad 100.00 g |

### Example 38: Leave in Conditioner not within the scope of the present invention

| | |
|---|---|
| Emulsion C | 1.80 g |
| vitamine E-acetate | 0.10 g |
| polymethylphenyl siloxane (CTFA: OUATERNIUM-80; Abil Quat^{(R)} 3272) | 0.50 g |
| propylene glycol | 10.00 g |
| behenyl trimethylammonium chloride | 0.50 g |
| sodium chloride | 0.05 g |
| d-panthenol | 0.30 g |
| PHB-propylester | 0.30 g |
| isododecane | 2.00 g |
| perfume oil | 0.20 g |
| water | ad 100.00 g |

### Example 39: Split Ends Fluid not within the scope of the present invention

| | |
|---|---|
| Emulsion A | 3.50 g |
| vitamine E-acetate | 0.10 g |
| polymethylphenyl siloxane (CTFA: OUATERNIUM-80; Abil Quat^{(R)} 3272) | 0.50 g |
| cyclo penta siloxane (CTFA: CYCLOMETHICONE) | 21.00 g |
| dihydroxy polydimethyl siloxane (CTFA: DIMETHICONOL) | 2.50 g |
| ethanol | 1.50 g |
| perfume oil | 0.60 g |
| water | ad 100.00 g |

### Example 40: Styling lotion

| | |
|---|---|
| Luviskol VA64 | 1.00 g |
| Emulsion C | 20.00 g |
| Eumulgin L | 0.20 g |
| Perfume | 0.15 g |
| PHENOXYETHANOL | 0.20 g |
| PHB-METHYLESTER | 0.12 g |
| DISODIUM EDTA | 0.10 g |
| Water | ad 100.00 g |

### Example 41: Styling gel

| | |
|---|---|
| PVP (LUVISKOL K 90 PULVER) | 2.00 g |
| NATROSOL 250 HHR | 0.50 g |
| Emulsion C | 20.00 g |
| Eumulgin L | 0.20 g |
| Perfume | 0.15 g |
| UVINUL P 25 | 0.10 g |
| PHENOXYETHANOL | 0.20 g |
| PHB-METHYLESTER | 0.12 g |
| DISODIUM EDTA | 0.10 g |
| Water | ad 100.00 g |

### Example 42: Aerosol Styling mousse

| | |
|---|---|
| Polyquaternium-11 (GAFQUAT 755 N) | 15.00 g |
| Emulsion C | 5.00 g |
| Laureth-4 | 0.40 g |
| Perfume | 0.15 g |
| PHENOXYETHANOL | 0.20 g |
| PHB-METHYLESTER | 0.12 g |
| DISODIUM EDTA | 0.10 g |
| Propane/Butane | 6.00 g |
| Water | ad 100.00 g |

### Example 43: Aerosol hairspray

| | |
|---|---|
| LUVISKOL VA 37 E | 8.00 g |
| Ethanol | 50.00 g |
| Surfactant 193 | 0.40 g |
| Perfume | 0.15 g |
| Eumulsion C | 5.00 g |
| Propane/Butane | 30.0 g |
| Water | ad 100.00 g |

### Example 44: Spray Gel

| | |
|---|---|
| Luviskol VA64 | 3.00 g |
| NATROSOL 250 HHR | 0.30 g |
| Emulsion C | 20.00 g |
| Eumulgin L | 0.20 g |
| Perfume | 0.15 g |
| PHENOXYETHANOL | 0.20 g |
| PHB-METHYLESTER | 0.12 g |
| DISODIUM EDTA | 0.10 g |
| Water | ad 100.00 g |

### Example 45: Leave-on Conditioner

| | |
|---|---|
| JAGUAR C-17 | 0.30 g |
| NATROSOL 250 HHR | 0.30 g |
| Emulsion C | 20.00 g |
| Eumulgin L | 0.20 g |
| Perfume | 0.15 g |
| PHENOXYETHANOL | 0.20 g |
| PHB-METHYLESTER | 0.12 g |
| DISODIUM EDTA | 0.10 g |
| Water | ad 100.00 g |

### Example 46: Shampoo

| | |
|---|---|
| JAGUAR C-162 | 0.20 g |
| SODIUM LAURETH SULFATE (LES 28%) | 40.00 g |
| COCAMIDOPROPYL BETAINE (TEGO BETAIN L 5045) | 5.00 g |
| DOW CORNING 200 FLUID/350 CS | 2.00 g |
| Perfume | 0.15 g |
| Emulsion C | 6.00 g |
| Water | ad 100.00 g |

### Example 47: Rinse-off Conditioner not within the scope of the present invention

| | |
|---|---|
| CETEARYL ALCOHOL | 4.50 g |
| CETRIMONIUM CHLORIDE (GENAMIN CTAC 50) | 1.30 g |
| Citric acid | 0.30 g |
| Perfume | 0.15 g |
| Emulsion C | 6.00 g |
| Water | ad 100.00 g |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. Use of a composition in the form of an emulsion, wherein the composition comprises:
(i) a polydimethylsiloxane (P) having aminoalkyl groups and having an amine number of at least 0.1 meq/g of polydimethylsiloxane (P), and comprising units of the formula I
R¹ₐR²_{b}SiO_{(4-a-b)/2} (I),
in which
R¹ are optionally halogen-substituted alkyl radicals having 1-40 carbon atoms, or are -OR or -OH radicals,
R are optionally halogen-substituted alkyl radicals having 1-40 carbon atoms,
R² are aminoalkyl radicals of the formula II
-R³-NR⁴R⁵ (II),
R³ are divalent hydrocarbon radicals having 1-40 carbon atoms,
R⁴ are monovalent hydrocarbon radicals having 1-40 carbon atoms or are H,
R⁵ is a radical of the formula III
-(R⁶-NR⁴)ₓR⁴ (III),
R⁶ is a divalent radical of the formula IV
-(CR⁴R⁴-)_{y} (IV),
x is 0 or a value from 1 to 40,
y is 1 or 2,
a is 0, 1, 2 or 3,
b is 0, 1, 2 or 3 and
a+b on average is from 1.5 to 2.5, not more than 9 mol % of the radical R¹ being OH or OR, and
(ii) a protonating agent,
for treating human hair, for decreasing the drying time for hair and simultaneously enhance the durability of a hair shape against hair humidity, water and washings;
wherein the protonating agent is formic acid, acetic acid, sulfuric acid, hydrochloric acid or citric acid;
wherein the composition contains auxiliaries which are selected from polyalcohols and ethers thereof, which have a boiling point or boiling range of not more than 260° C at 0.10 MPa;
and wherein the composition contains not more than 3 parts by weight of emulsifier or surfactant.

2. Use according to claim 1, wherein the "treating" is making the hair water repellent.

3. Use according to any one of claims 1 to 2, for reducing the drying time of human hair on the head of a person.

4. Use according to any one of claims 1 to 3, for reducing the drying time of human hair and simultaneously protecting the hair dye of a previously dyed hair from being washed out.

5. Use according to claim 1, for reducing the time to dry wet hair and also styling the hair.

6. Use according to claim 1, for treating hair, preferably for styling hair, which makes the treated hair water repellent even after one wash, preferably even after more than 2 washes.

7. Use according to any of the preceding claims, wherein the composition comprises at least one pigment, wherein the pigments are preferably present in undissolved form and are present in an amount of from 0.01 to 25% by weight.

8. Use according to claim 7, wherein the pigments have a particle size of 1 to 200 µm, in particular 3 to 150 µm, particularly preferably 10 to 100 µm.

9. Use according to any of claims 7-8, wherein the pigments are selected from white pigments, such as-titanium dioxide or zinc oxide, black pigments, such as iron oxide black, colored pigments, such as, ultramarine or iron oxide red, luster pigments, metal effect pigments, pearlescent pigments, and fluorescent or phosphorescent pigments, where preferably at least one pigment is a colored, nonwhite pigment.

10. Use according to claims 7-9, wherein the pigments are selected from pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine.

11. A hair treating composition, in the form of an emulsion, comprising a combination of:
(A) a polydimethylsiloxane (P) having aminoalkyl groups and having an amine number of at least 0.1 meq/g of polydimethylsiloxane (P), and comprising units of the formula I
R¹ₐR²_{b}SiO_{(4-a-b)/2} (I),
in which
R¹ are optionally halogen-substituted alkyl radicals having 1-40 carbon atoms, or are -OR or -OH radicals,
R are optionally halogen-substituted alkyl radicals having 1-40 carbon atoms,
R² are aminoalkyl radicals of the formula II
-R³-NR⁴R⁵ (II),
R³ are divalent hydrocarbon radicals having 1-40 carbon atoms,
R⁴ are monovalent hydrocarbon radicals having 1-40 carbon atoms or are H,
R⁵ is a radical of the formula III
-(R⁶-NR⁴)ₓR⁴ (III),
R⁶ is a divalent radical of the formula IV
-(CR⁴R⁴-)_{y} (IV),
x is 0 or a value from 1 to 40,
y is 1 or 2,
a is 0, 1, 2 or 3,
b is 0, 1, 2 or 3 and
a+b on average is from 1.5 to 2.5, not more than 9 mol % of the radical R¹ being OH or OR,
(B) at least one protonating agent,
(C) water,
(D) from 0 to 95 parts by weight of at least one emulsifier and/or surfactant, and
(E) at least one cosmetic hair treatment agent, selected from the group consisting of hair styling polymers or, hair styling polymers and hair conditioning agents,
wherein the protonating agent is formic acid, acetic acid, sulfuric acid, hydrochloric acid or citric acid;
wherein the composition contains auxiliaries which are selected from polyalcohols and ethers thereof, which have a boiling point or boiling range of not more than 260° C at 0.10 MPa;
and wherein the composition contains not more than 3 parts by weight of emulsifier or surfactant.

12. A composition according to claim 11, in form of an oil-in-water emulsion.

13. A composition according to any one of claims 11 or 12, wherein the polydimethylsiloxane (P) is present in an amount of from 0.001 to 50 % by weight of the total weight of the hair treating composition.

14. A composition according to any one of claims 11 to 13, wherein the composition comprises at least one pigment, wherein the pigments are preferably present in undissolved form and are present in an amount of from 0.01 to 25% by weight.

15. A composition according to claim 14, wherein the pigments have a particle size of 1 to 200 µm, in particular 3 to 150 µm, particularly preferably 10 to 100 µm.

16. A composition according to any one of claims 11 to 15, wherein the pigments are selected from white pigments, such as titanium dioxide or zinc oxide, black pigments, such as iron oxide black, colored pigments, such as, ultramarine or iron oxide red, luster pigments, metal effect pigments, pearlescent pigments, and fluorescent or phosphorescent pigments, where preferably at least one pigment is a colored, nonwhite pigment.

17. A composition according to any one of claims 11 to 16, wherein the pigments are selected from pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine.

18. A method for treating human hair on the scalp of a person while simultaneously reducing the time to dry the hair, comprising the steps of applying to dry or wet hair a composition of claim 11 and subsequently drying the hair.

19. A method for styling human hair on the scalp of a person while simultaneously reducing the time to dry the hair, comprising the steps of applying to dry or wet hair a composition of claim 11 and subsequently drying the hair.

20. A method according to claim 19, whereby before or during the drying step, the hair is mechanically processed to help water to sheet from the hair by brushing, combing or towelling.

21. A method for dying the hair on the head of a person while simultaneously reducing the time to dry said hair and to protect the hair dye on said hair from being washed out, comprising the steps of:
(a) applying a hair dying composition onto the hair to be dyed,
(b) leaving the hair dying composition on the hair for a time required to dye said hair,
(c) optionally rinsing said hair with water or an aqueous acidic composition,
(d) optionally towelling and/or drying said hair,
(e) applying to said hair a composition as used according to claim 1 or a hair treating composition, in the form of an emulsion, comprising a combination of:
(A) a polydimethylsiloxane (P) having aminoalkyl groups and having an amine number of at least 0.1 meq/g of polydimethylsiloxane (P), and comprising units of the formula I
R¹_{A}R²_{b}SiO_{(4-a-b)/2} (I),
in which
R¹ are optionally halogen-substituted alkyl radicals having 1-40 carbon atoms, or are - OR or -OH radicals,
R are optionally halogen-substituted alkyl radicals having 1-40 carbon atoms,
R² are aminoalkyl radicals of the formula II
-R³-NR⁴R⁵ (II),
R³ are divalent hydrocarbon radicals having 1-40 carbon atoms,
R⁴ are monovalent hydrocarbon radicals having 1-40 carbon atoms or are H,
R⁵ is a radical of the formula III
-(R⁶-NR⁴)ₓR⁴ (III),
R⁶ is a divalent radical of the formula IV
-(CR⁴R⁴-)_{y} (IV),
x is 0 or a value from 1 to 40,
y is 1 or 2,
a is 0, 1, 2 or 3,
b is 0, 1, 2 or 3 and
a-b on average is from 1.5 to 2.5. not more than 9 mol % of the radical R¹ being OH or OR.
(B) at least one protonating agent,
(C) water,
(D) from 0 to 95 parts by weight of at least one emulsifier and/or surfactant, and
(E) at least one cosmetic hair treatment agent, selected from the group consisting of hair styling polymers and/or hair conditioning agents,
wherein the protonating agent is formic acid, acetic acid, sulfuric acid, hydrochloric acid or citric acid;
wherein the composition contains auxiliaries which are selected from polyalcohols and ethers thereof, which have a boiling point or boiling range of not more than 260° C at 0.10 MPa;
and wherein the composition contains not more than 3 parts by weight of emulsifier or surfactant and
(f) subsequently drying said hair.

22. A method for enhancing the durability of a hair shape against humidity, water and hair washings while simultaneously reducing the time to dry said hair, comprising the steps of:
(a) shaping the hair,
(b) optionally rinsing said hair with water or an aqueous acidic composition,
(c) optionally towelling and/or drying said hair,
(d) applying to said hair a composition as used according to claim 1 or a hair treating composition according to claim 11 and
(e) subsequently drying said hair.

23. A method for enhancing the durability of a hair shape against humidity, water and hair washings while simultaneously reducing the time to dry said hair, comprising the steps of:
(a) washing said hair,
(b) optionally towelling and/or drying said hair,
(c) applying to said hair a composition as used according to claim 1 or a hair treating composition according to claim 11 and
(d) subsequently drying and simultaneously shaping said hair.

## Patentansprüche

1. Verwendung einer Zusammensetzung in Form einer Emulsion, wobei die Zusammensetzung Folgendes umfasst:
(i) ein Polydimethylsiloxan (P), aufweisend Aminoalkylgruppen und aufweisend eine Aminzahl von mindestens 0,1 meq/g Polydimethylsiloxan (P), und umfassend Einheiten der Formel I
R¹ₐR²_{b}SiO_{(4-a-b)/2} (I),
worin
R¹ wahlweise halogensubstituierte Alkylreste mit 1-40 Kohlenstoffatomen sind oder -OR- oder -OH-Reste sind,
R wahlweise halogensubstituierte Alkylreste mit 1-40 Kohlenstoffatomen sind,
R² Aminoalkylreste der Formel II
-R³-NR⁴R⁵ (II)
sind,
R³ zweiwertige Kohlenwasserstoffreste mit 1-40 Kohlenstoffatomen sind,
R⁴ einwertige Kohlenwasserstoffreste mit 1-40 Kohlenstoffatomen sind oder H sind,
R⁵ ein Rest der Formel III
-(R⁶-NR⁴)ₓR⁴ (III)
ist,
R⁶ ein zweiwertiger Rest der Formel IV
-(CR⁴R⁴-)_{y} (IV)
ist,
x 0 oder ein Wert von 1 bis 40 ist,
y 1 oder 2 ist,
a 0, 1, 2 oder 3 ist,
b 0, 1, 2 oder 3 ist und
a+b durchschnittlich von 1,5 bis 2,5 ist, wobei nicht mehr als 9 Mol-% des Rests R¹ OH oder OR sind, und
(ii) ein Protonierungsmittel,
zum Behandeln von menschlichem Haar zum Verringern der Haartrocknungszeit und gleichzeitigem Erhöhen der Haltbarkeit einer Haarform gegenüber Haarfeuchtigkeit, Wasser und Wäschen;
wobei das Protonierungsmittel Ameisensäure, Essigsäure, Schwefelsäure, Salzsäure oder Citronensäure ist;
wobei die Zusammensetzung Hilfsmittel enthält, die ausgewählt sind aus Polyalkoholen und Ethern davon, die einen Siedepunkt oder Siedebereich von nicht mehr als 260 °C bei 0,10 MPa aufweisen;
und wobei die Zusammensetzung nicht mehr als 3 Gewichtsteile Emulgator oder Tensid enthält.

2. Verwendung nach Anspruch 1, wobei das "Behandeln" bedeutet, das Haar wasserabweisend zu machen.

3. Verwendung nach einem der Ansprüche 1 bis 2 zum Verringern der Trocknungszeit von menschlichem Haar auf dem Kopf einer Person.

4. Verwendung nach einem der Ansprüche 1 bis 3 zum Verringern der Trocknungszeit von menschlichem Haar und gleichzeitigem Schützen der Haarfarbe von zuvor gefärbtem Haar vor Auswaschen.

5. Verwendung nach Anspruch 1 zum Verringern der Zeit zum Trocknen von nassem Haar und zudem Frisieren des Haars.

6. Verwendung nach Anspruch 1 zum Behandeln von Haar, vorzugsweise zum Frisieren von Haar, wodurch das behandelte Haar selbst nach einer einzelnen Wäsche, vorzugsweise selbst nach mehr als 2 Wäschen, wasserabweisend wird.

7. Verwendung nach einem der vorstehenden Ansprüche, wobei die Zusammensetzung mindestens ein Pigment umfasst, wobei die Pigmente vorzugsweise in ungelöster Form vorhanden sind und in einer Menge von 0,01 bis 25 Gew.-% vorhanden sind.

8. Verwendung nach Anspruch 7, wobei die Pigmente eine Teilchengröße von 1 bis 200 µm, insbesondere 3 bis 150 µm, insbesondere bevorzugt 10 bis 100 µm aufweisen.

9. Verwendung nach einem der Ansprüche 7 bis 8, wobei die Pigmente ausgewählt sind aus weißen Pigmenten, wie Titandioxid oder Zinkoxid, schwarzen Pigmenten, wie Eisenoxidschwarz, farbigen Pigmenten, wie Ultramarin oder Eisenoxidrot, Glanzpigmenten, Metalleffektpigmenten, Perlglanzpigmenten und fluoreszierenden oder phosphoreszierenden Pigmenten, wobei vorzugsweise mindestens ein Pigment ein farbiges, nichtweißes Pigment ist.

10. Verwendung nach den Ansprüchen 7 bis 9, wobei die Pigmente ausgewählt sind aus Perlglanz- und farbigen Pigmenten auf Basis von Glimmer, die mit einem Metalloxid oder einem Metalloxidchlorid, wie Titandioxid oder Bismutoxidchlorid, beschichtet sind, und wahlweise ferner farbverleihenden Substanzen, wie Eisenoxiden, Preußischblau, Ultramarin, Karmin.

11. Haarbehandlungszusammensetzung in Form einer Emulsion, umfassend eine Kombination aus:
(A) einem Polydimethylsiloxan (P), aufweisend Aminoalkylgruppen und aufweisend eine Aminzahl von mindestens 0,1 meq/g Polydimethylsiloxan (P), und umfassend Einheiten der Formel I
R¹ₐR²_{b}SiO_{(4-a-b)/2} (I),
worin
R¹ wahlweise halogensubstituierte Alkylreste mit 1-40 Kohlenstoffatomen sind oder -OR- oder -OH-Reste sind,
R wahlweise halogensubstituierte Alkylreste mit 1-40 Kohlenstoffatomen sind,
R² Aminoalkylreste der Formel II
-R³-NR⁴R⁵ (II)
sind,
R³ zweiwertige Kohlenwasserstoffreste mit 1-40 Kohlenstoffatomen sind,
R⁴ einwertige Kohlenwasserstoffreste mit 1-40 Kohlenstoffatomen sind oder H sind,
R⁵ ein Rest der Formel III
-(R⁶-NR⁴)ₓR⁴ (III)
ist,
R⁶ ein zweiwertiger Rest der Formel IV
-(CR⁴R⁴-)_{y} (IV)
ist,
x 0 oder ein Wert von 1 bis 40 ist,
y 1 oder 2 ist,
a 0, 1, 2 oder 3 ist,
b 0, 1, 2 oder 3 ist und
a+b durchschnittlich von 1,5 bis 2,5 ist, wobei nicht mehr als 9 Mol-% des Rests R¹ OH oder OR sind,
(B) mindestens einem Protonierungsmittel,
(C) Wasser,
(D) von 0 bis 95 Gewichtsteilen mindestens eines Emulgators und/oder Tensids und
(E) mindestens einem kosmetischen Haarbehandlungsmittel, ausgewählt aus der Gruppe bestehend aus Haarfrisierpolymeren oder Haarfrisierpolymeren und Haarkonditioniermitteln,
wobei das Protonierungsmittel Ameisensäure, Essigsäure, Schwefelsäure, Salzsäure oder Citronensäure ist;
wobei die Zusammensetzung Hilfsmittel enthält, die ausgewählt sind aus Polyalkoholen und Ethern davon, die einen Siedepunkt oder Siedebereich von nicht mehr als 260 °C bei 0,10 MPa aufweisen;
und wobei die Zusammensetzung nicht mehr als 3 Gewichtsteile Emulgator oder Tensid enthält.

12. Zusammensetzung nach Anspruch 11, in Form einer Öl-in-Wasser-Emulsion.

13. Zusammensetzung nach einem der Ansprüche 11 oder 12, wobei das Polydimethylsiloxan (P) in einer Menge von 0,001 bis 50 Gew.-% des Gesamtgewichts der Haarbehandlungszusammensetzung vorhanden ist.

14. Zusammensetzung nach einem der Ansprüche 11 bis 13, wobei die Zusammensetzung mindestens ein Pigment umfasst, wobei die Pigmente vorzugsweise in ungelöster Form vorhanden sind und in einer Menge von 0,01 bis 25 Gew.-% vorhanden sind.

15. Zusammensetzung nach Anspruch 14, wobei die Pigmente eine Teilchengröße von 1 bis 200 µm, insbesondere 3 bis 150 µm, insbesondere bevorzugt 10 bis 100 µm aufweisen.

16. Zusammensetzung nach einem der Ansprüche 11 bis 15, wobei die Pigmente ausgewählt sind aus weißen Pigmenten, wie Titandioxid oder Zinkoxid, schwarzen Pigmenten, wie Eisenoxidschwarz, farbigen Pigmenten, wie Ultramarin oder Eisenoxidrot, Glanzpigmenten, Metalleffektpigmenten, Perlglanzpigmenten und fluoreszierenden oder phosphoreszierenden Pigmenten, wobei vorzugsweise mindestens ein Pigment ein farbiges, nichtweißes Pigment ist.

17. Zusammensetzung nach einem der Ansprüche 11 bis 16, wobei die Pigmente ausgewählt sind aus Perlglanz- und farbigen Pigmenten auf Basis von Glimmer, die mit einem Metalloxid oder einem Metalloxidchlorid, wie Titandioxid oder Bismutoxidchlorid, beschichtet sind, und wahlweise ferner farbverleihenden Substanzen, wie Eisenoxiden, Preußischblau, Ultramarin, Karmin.

18. Verfahren zum Behandeln von menschlichem Haar auf der Kopfhaut einer Person bei gleichzeitigem Verringern der Zeit zum Trocknen des Haars, umfassend die Schritte des Auftragens einer Zusammensetzung nach Anspruch 11 auf trockenes oder nasses Haar und des anschließenden Trocknens des Haars.

19. Verfahren zum Frisieren von menschlichem Haar auf der Kopfhaut einer Person bei gleichzeitigem Verringern der Zeit zum Trocknen des Haars, umfassend die Schritte des Auftragens einer Zusammensetzung nach Anspruch 11 auf trockenes oder nasses Haar und des anschließenden Trocknens des Haars.

20. Verfahren nach Anspruch 19, wobei vor oder während des Trocknungsschritts das Haar durch Bürsten, Kämmen oder Abtrocknen mit einem Handtuch mechanisch bearbeitet wird, um das Ablaufen des Wassers aus dem Haar zu unterstützen.

21. Verfahren zum Trocknen des Haars auf dem Kopf einer Person bei gleichzeitiger Verringerung der Zeit zum Trocknen des Haars und zum Schützen der Haarfarbe auf dem Haar vor Auswaschen, umfassend die Schritte:
(a) Auftragen einer Haarfärbezusammensetzung auf das zu färbende Haar,
(b) Belassen der Haarfärbezusammensetzung auf dem Haar für eine Zeitdauer, die zum Färben des Haars erforderlich ist,
(c) wahlweise Spülen des Haars mit Wasser oder einer wässrigen sauren Zusammensetzung,
(d) wahlweise Abtrocknen des Haars mit einem Handtuch und/oder Trocknen des Haars,
(e) Auftragen einer Zusammensetzung, wie nach Anspruch 1 verwendet, oder einer Haarbehandlungszusammensetzung, in Form einer Emulsion, auf das Haar, umfassend eine Kombination aus:
(A) einem Polydimethylsiloxan (P), aufweisend Aminoalkylgruppen und aufweisend eine Aminzahl von mindestens 0,1 meq/g Polydimethylsiloxan (P), und umfassend Einheiten der Formel I
R¹ₐR²_{b}SiO_{(4-a-b)/2} (I),
worin
R¹ wahlweise halogensubstituierte Alkylreste mit 1-40 Kohlenstoffatomen sind oder -OR- oder -OH-Reste sind,
R wahlweise halogensubstituierte Alkylreste mit 1-40 Kohlenstoffatomen sind,
R² Aminoalkylreste der Formel II
-R³-NR⁴R⁵ (II)
sind,
R³ zweiwertige Kohlenwasserstoffreste mit 1-40 Kohlenstoffatomen sind,
R⁴ einwertige Kohlenwasserstoffreste mit 1-40 Kohlenstoffatomen sind oder H sind,
R⁵ ein Rest der Formel III
-(R⁶-NR⁴)ₓR⁴ (III)
ist,
R⁶ ein zweiwertiger Rest der Formel IV
-(CR⁴R⁴-)_{y} (IV)
ist,
x 0 oder ein Wert von 1 bis 40 ist,
y 1 oder 2 ist,
a 0, 1, 2 oder 3 ist,
b 0, 1, 2 oder 3 ist und
a+b durchschnittlich von 1,5 bis 2,5 ist, wobei nicht mehr als 9 Mol-% des Rests R¹ OH oder OR sind,
(B) mindestens einem Protonierungsmittel,
(C) Wasser,
(D) von 0 bis 95 Gewichtsteilen mindestens eines Emulgators und/oder Tensids, und
(E) mindestens einem kosmetischen Haarbehandlungsmittel, ausgewählt aus der Gruppe bestehend aus Haarfrisierpolymeren und/oder Haarkonditioniermitteln,
wobei das Protonierungsmittel Ameisensäure, Essigsäure, Schwefelsäure, Salzsäure oder Citronensäure ist;
wobei die Zusammensetzung Hilfsmittel enthält, die ausgewählt sind aus Polyalkoholen und Ethern davon, die einen Siedepunkt oder Siedebereich von nicht mehr als 260 °C bei 0,10 MPa aufweisen;
und wobei die Zusammensetzung nicht mehr als 3 Gewichtsteile Emulgator oder Tensid enthält, und
(f) anschließendes Trocknen des Haars.

22. Verfahren zum Erhöhen der Haltbarkeit einer Haarform gegenüber Feuchtigkeit, Wasser und Haarwäschen bei gleichzeitigem Verringern der Zeit zum Trocknen des Haars, umfassend die Schritte:
(a) Formen des Haars,
(b) wahlweise Spülen des Haars mit Wasser oder einer wässrigen sauren Zusammensetzung,
(c) wahlweise Abtrocknen des Haars mit einem Handtuch und/oder Trocknen des Haars,
(d) Auftragen einer Zusammensetzung, wie nach Anspruch 1 verwendet, oder einer Haarbehandlungszusammensetzung nach Anspruch 11 auf das Haar und
(e) anschließendes Trocknen des Haars.

23. Verfahren zum Erhöhen der Haltbarkeit einer Haarform gegenüber Feuchtigkeit, Wasser und Haarwäschen bei gleichzeitigem Verringern der Zeit zum Trocknen des Haars, umfassend die Schritte:
(a) Waschen des Haars,
(b) wahlweise Abtrocknen des Haars mit einem Handtuch und/oder Trocknen des Haars,
(c) Auftragen einer Zusammensetzung, wie nach Anspruch 1 verwendet, oder einer Haarbehandlungszusammensetzung nach Anspruch 11 auf das Haar und
(d) anschließendes Trocknen und gleichzeitiges Formen des Haars.

## Revendications

1. Utilisation d'une composition sous la forme d'une émulsion, dans laquelle la composition comprend :
(i) un polydiméthylsiloxane (P) ayant des groupes aminoalkyle et ayant un indice d'amine d'au moins 0,1 méq/g de polydiméthylsiloxane (P), et comprenant des motifs de formule I
R¹ₐR²_{b}SiO_{(4-a-b)/2} (I),
dans laquelle
les R¹ sont des radicaux alkyle facultativement substitués par un halogène, ayant 1 à 40 atomes de carbone, ou sont des radicaux -OR ou -OH,
les R sont des radicaux alkyle facultativement substitués par un halogène, ayant 1 à 40 atomes de carbone,
les R² sont des radicaux aminoalkyle de formule II
-R³-NR⁴R⁵ (II),
les R³ sont des radicaux hydrocarbonés divalents ayant 1 à 40 atomes de carbone,
les R⁴ sont des radicaux hydrocarbonés monovalents ayant 1 à 40 atomes de carbone ou sont H,
R⁵ est un radical de formule III
-(R⁶-NR⁴)ₓR⁴ (III),
R⁶ est un radical divalent de formule IV
x vaut 0 ou une valeur allant de 1 à 40,
y vaut 1 ou 2,
a vaut 0, 1, 2 ou 3,
b vaut 0, 1, 2 ou 3 et
a+b en moyenne va de 1,5 à 2,5, pas plus de 9 % molaires du radical R¹ étant OH ou OR, et
(ii) un agent de protonation,
pour traiter des cheveux humains, pour diminuer le temps de séchage pour les cheveux et améliorer simultanément la durabilité d'une mise en forme de cheveux vis-à-vis de l'humidité des cheveux, de l'eau et des lavages ;
dans laquelle l'agent de protonation est l'acide formique, l'acide acétique, l'acide sulfurique, l'acide chlorhydrique ou l'acide citrique ;
dans laquelle la composition contient des agents auxiliaires qui sont choisis parmi des polyalcools et éthers de ceux-ci, qui ont un point d'ébullition ou une plage d'ébullition de pas plus de 260 °C à 0,10 MPa ;
et dans laquelle la composition ne contient pas plus de 3 parties en poids d'émulsifiant ou d'agent tensioactif.

2. Utilisation selon la revendication 1, dans laquelle le « traitement » consiste à rendre les cheveux hydrofuges.

3. Utilisation selon l'une quelconque des revendications 1 à 2, pour réduire le temps de séchage des cheveux humains sur la tête d'une personne.

4. Utilisation selon l'une quelconque des revendications 1 à 3, pour réduire le temps de séchage des cheveux humains et protéger simultanément la teinture capillaire de cheveux précédemment teints vis-à-vis d'une élimination par lavage.

5. Utilisation selon la revendication 1, pour réduire le temps pour sécher des cheveux mouillés et également pour coiffer les cheveux.

6. Utilisation selon la revendication 1, pour le traitement des cheveux, de préférence pour le coiffage des cheveux, qui rend les cheveux traités hydrofuges même après un lavage, de préférence même après plus de 2 lavages.

7. Utilisation selon l'une quelconque des revendications précédentes, dans laquelle la composition comprend au moins un pigment, dans laquelle les pigments sont, de préférence, présents sous forme non dissoute et sont présents en une quantité de 0,01 à 25 % en poids.

8. Utilisation selon la revendication 7, dans laquelle les pigments ont une taille de particules de 1 à 200 µm, en particulier 3 à 150 µm, en particulier de préférence de 10 à 100 µm.

9. Utilisation selon l'une quelconque des revendications 7 à 8, dans laquelle les pigments sont choisis parmi des pigments blancs, tels que le dioxyde de titane ou l'oxyde de zinc, des pigments noirs, tels que le noir d'oxyde de fer, des pigments colorés, tels que le bleu d'outre-mer ou le rouge d'oxyde de fer, des pigments d'éclat, des pigments à effet métallique, des pigments nacrés et des pigments fluorescents ou phosphorescents, où de préférence au moins un pigment est un pigment coloré, non blanc.

10. Utilisation selon les revendications 7 à 9, dans laquelle les pigments sont choisis parmi des pigments nacrés et colorés à base de mica, qui sont revêtus d'un oxyde métallique ou d'un oxychlorure métallique, tel que le dioxyde de titane ou l'oxychlorure de bismuth, et facultativement en outre des substances communiquant de la couleur, telles que des oxydes de fer, du bleu de Prusse, du bleu d'outre-mer, du carmin.

11. Composition de traitement des cheveux, sous la forme d'une émulsion, comprenant une combinaison de :
(A) un polydiméthylsiloxane (P) ayant des groupes aminoalkyle et ayant un indice d'amine d'au moins 0,1 méq/g de polydiméthylsiloxane (P), et comprenant des motifs de formule I
R¹ₐR²_{b}SiO_{(4-a-b)/2} (I),
dans laquelle
les R¹ sont des radicaux alkyle facultativement substitués par un halogène, ayant 1 à 40 atomes de carbone, ou sont des radicaux -OR ou -OH,
les R sont des radicaux alkyle facultativement substitués par un halogène, ayant 1 à 40 atomes de carbone,
les R² sont des radicaux aminoalkyle de formule II
-R³-NR⁴R⁵ (II),
les R³ sont des radicaux hydrocarbonés divalents ayant 1 à 40 atomes de carbone,
les R⁴ sont des radicaux hydrocarbonés monovalents ayant 1 à 40 atomes de carbone ou sont H,
R⁵ est un radical de formule III
-(R⁶-NR⁴)ₓR⁴ (III),
R⁶ est un radical divalent de formule IV
-(CR⁴R⁴-)_{y} (IV),
x vaut 0 ou une valeur allant de 1 à 40,
y vaut 1 ou 2,
a vaut 0, 1, 2 ou 3,
b vaut 0, 1, 2 ou 3 et
a·b en moyenne va de 1,5 à 2,5, pas plus de 9 % molaires du radical R¹ étant OH ou OR,
(B) au moins un agent de protonation,
(C) de l'eau,
(D) de 0 à 95 parties en poids d'au moins un émulsifiant et/ou agent tensioactif, et
(E) au moins un agent traitement capillaire cosmétique, choisi dans le groupe constitué de polymères de coiffage de cheveux ou de polymères de coiffage de cheveux et agents de conditionnement des cheveux,
dans laquelle l'agent de protonation est l'acide formique, l'acide acétique, l'acide sulfurique, l'acide chlorhydrique ou l'acide citrique ;
dans laquelle la composition contient des agents auxiliaires qui sont choisis parmi des polyalcools et éthers de ceux-ci, qui ont un point d'ébullition ou une plage d'ébullition de pas plus de 260 °C à 0,10 MPa ;
et dans laquelle la composition ne contient pas plus de 3 parties en poids d'émulsifiant ou d'agent tensioactif.

12. Composition selon la revendication 11, sous forme d'une émulsion huile-dans-eau.

13. Composition selon l'une quelconque des revendications 11 ou 12, dans laquelle le polydiméthylsiloxane (P) est présent en une quantité de 0,001 à 50 % en poids du poids total de la composition de traitement des cheveux.

14. Composition selon l'une quelconque des revendications 11 à 13, où la composition comprend au moins un pigment, dans laquelle les pigments sont, de préférence, présents sous forme non dissoute et sont présents en une quantité de 0,01 à 25 % en poids.

15. Composition selon la revendication 14, dans laquelle les pigments ont une taille de particules de 1 à 200 µm, en particulier 3 à 150 µm, en particulier de préférence de 10 à 100 µm.

16. Composition selon l'une quelconque des revendications 11 à 15, dans laquelle les pigments sont choisis parmi des pigments blancs, tels que le dioxyde de titane ou l'oxyde de zinc, des pigments noirs, tels que le noir d'oxyde de fer, des pigments colorés, tels que le bleu d'outre-mer ou le rouge d'oxyde de fer, des pigments d'éclat, des pigments à effet métallique, des pigments nacrés et des pigments fluorescents ou phosphorescents, où de préférence au moins un pigment est un pigment coloré, non blanc.

17. Composition selon l'une quelconque des revendications 11 à 16, dans laquelle les pigments sont choisis parmi des pigments nacrés et colorés à base de mica, qui sont revêtus d'un oxyde métallique ou d'un oxychlorure métallique, tel que le dioxyde de titane ou l'oxychlorure de bismuth, et facultativement en outre des substances communiquant de la couleur, telles que des oxydes de fer, du bleu de Prusse, du bleu d'outre-mer, du carmin.

18. Procédé pour traiter des cheveux humains sur le cuir chevelu d'une personne tout en réduisant simultanément le temps pour sécher les cheveux, comprenant les étapes consistant à appliquer sur des cheveux secs ou mouillés une composition selon la revendication 11, et à sécher ensuite les cheveux.

19. Procédé pour coiffer des cheveux humains sur le cuir chevelu d'une personne tout en réduisant simultanément le temps pour sécher les cheveux, comprenant les étapes consistant à appliquer sur des cheveux secs ou mouillés une composition selon la revendication 11, et à sécher ensuite les cheveux.

20. Procédé selon la revendication 19, selon lequel, avant ou pendant l'étape de séchage, les cheveux sont traités mécaniquement pour aider l'eau à s'écouler des cheveux par brossage, peignage ou essuyage.

21. Procédé de teinture des cheveux sur la tête d'une personne tout en réduisant simultanément le temps pour sécher lesdits cheveux et pour protéger la teinture capillaire sur lesdits cheveux d'une élimination par lavage, comprenant les étapes consistant à :
(a) appliquer une composition de teinture capillaire sur les cheveux à teindre,
(b) laisser la composition de teinture capillaire sur les cheveux pendant un temps nécessaire pour teindre lesdits cheveux,
(c) facultativement rincer lesdits cheveux avec de l'eau ou une composition acide aqueuse,
(d) facultativement essuyer et/ou sécher lesdits cheveux,
(e) appliquer sur lesdits cheveux une composition telle qu'utilisée selon la revendication 1 ou une composition de traitement des cheveux, sous la forme d'une émulsion, comprenant une combinaison de :
(A) un polydiméthylsiloxane (P) ayant des groupes aminoalkyle et ayant un indice d'amine d'au moins 0,1 méq/g de polydiméthylsiloxane (P), et comprenant des motifs de formule I
R¹ₐR²_{b}SiO_{(4-a-b)}/₂ (I),
dans laquelle
les R¹ sont des radicaux alkyle facultativement substitués par un halogène, ayant 1 à 40 atomes de carbone, ou sont des radicaux -OR ou -OH,
les R sont des radicaux alkyle facultativement substitués par un halogène, ayant 1 à 40 atomes de carbone,
les R² sont des radicaux aminoalkyle de formule II
-R³-NR⁴R⁵ (II),
les R³ sont des radicaux hydrocarbonés divalents ayant 1 à 40 atomes de carbone,
les R⁴ sont des radicaux hydrocarbonés monovalents ayant 1 à 40 atomes de carbone ou sont H,
R⁵ est un radical de formule III
-(R⁶-NR⁴)ₓR⁴ (III),
R⁶ est un radical divalent de formule IV
-(CR⁴R⁴-)_{y} (IV),
x vaut 0 ou une valeur allant de 1 à 40,
y vaut 1 ou 2,
a vaut 0, 1, 2 ou 3,
b vaut 0, 1, 2 ou 3 et
a·b en moyenne va de 1,5 à 2,5, pas plus de 9 % molaires du radical R¹ étant OH ou OR,
(B) au moins un agent de protonation,
(C) de l'eau,
(D) de 0 à 95 parties en poids d'au moins un émulsifiant et/ou agent tensioactif, et
(E) au moins un agent traitement capillaire cosmétique, choisi dans le groupe constitué de polymères de coiffage des cheveux et/ou d'agents de conditionnement des cheveux,
dans lequel l'agent de protonation est l'acide formique, l'acide acétique, l'acide sulfurique, l'acide chlorhydrique ou l'acide citrique ;
dans lequel la composition contient des agents auxiliaires qui sont choisis parmi des polyalcools et éthers de ceux-ci, qui ont un point d'ébullition ou une plage d'ébullition de pas plus de 260 °C à 0,10 MPa ;
et dans lequel la composition ne contient pas plus de 3 parties en poids d'émulsifiant ou d'agent tensioactif et
(f) sécher ensuite lesdits cheveux.

22. Procédé pour renforcer la durabilité d'une mise en forme de cheveux vis-à-vis de l'humidité, de l'eau et des lavages de cheveux tout en réduisant simultanément le temps pour sécher lesdits cheveux, comprenant les étapes consistant à :
(a) mettre en forme les cheveux,
(b) facultativement rincer lesdits cheveux avec de l'eau ou une composition acide aqueuse,
(c) facultativement essuyer et/ou sécher lesdits cheveux,
(d) appliquer auxdits cheveux une composition telle qu'utilisée selon la revendication 1 ou une composition de traitement des cheveux selon la revendication 11 et
(e) sécher ensuite lesdits cheveux.

23. Procédé pour renforcer la durabilité d'une mise en forme de cheveux vis-à-vis de l'humidité, de l'eau et des lavages de cheveux tout en réduisant simultanément le temps pour sécher lesdits cheveux, comprenant les étapes consistant à :
(a) laver lesdits cheveux,
(b) facultativement essuyer et/ou sécher lesdits cheveux,
(c) appliquer auxdits cheveux une composition telle qu'utilisée selon la revendication 1 ou une composition de traitement des cheveux selon la revendication 11 et
(d) sécher ensuite et mettre en forme simultanément lesdits cheveux.
